# FASCICULE DE BREVET EUROPEEN

(11) **EP 0 784 693 B1**
(45) Date de publication et mention de la délivrance du brevet: **04.10.2001**
(21) Numéro de dépôt: 96926455.5
(22) Date de dépôt: 24.07.1996
(51) Int. Cl.: C12N 15/86, C12N 7/01, A61K 48/00, A61K 39/235

(54) **VECTEURS VIRAUX POUR LA THERAPIE GENIQUE**
VIRALE VEKTOREN FÜR DIE GENTHERAPIE
VIRAL VECTORS FOR GENE THERAPY

(30) Priorité: 24.07.1995 FR 9508946; 09.04.1996 FR 9604413
(43) Date de publication de la demande: 23.07.1997
(60) Demande divisionnaire: 00128382.9
(73) Titulaire: TRANSGENE S.A., 67000 Strasbourg (FR)
(72) Inventeur: MEHTALI, Majid, F-67400 Illkirch-Graffenstaden (FR); LUSKY, Monika, D-07910 Freiburg (DE); RITTNER, Karola, F-67000 Strasbourg (FR)
(74) Mandataire: Warcoin, Jacques
(86) Numéro de dépôt international: FR9601165
(87) Numéro de publication internationale: WO9704119

(56) Documents cités:
- WO-A-94/28152
- WO-A-95/02697
- WO-A-96/14061
- WO-A-97/00947
- GENE THER, MAR 1995, 2 (2) P124-315, ENGLAND, XP000568345 HERSH J ET AL: "Modulation of gene expression after replication-deficient, recombinant adenovirus-mediated gene transfer by the product of a second adenovirus vector."
- J VIROL, APR 1995, 69 (4) P2565-73, UNITED STATES, XP002002114 KIM HJ ET AL: "Tetracycline repressor-regulated gene repression in recombinant human cytomegalovirus." cité dans la demande
- MOL. CELL. BIOL. (APR 1995), 15(4), 1907-14 CODEN: MCEBD4;ISSN: 0270-7306, XP000564650 DEUSCHLE, ULRICH ET AL: "Tetracycline-reversible silencing of eukaryotic promoters"
- NUCLEIC ACIDS RES. (1991), 19(23), 6579-86 CODEN: NARHAD;ISSN: 0305-1048, XP000604601 O'CONNOR, ROBERT J. ET AL: "The C-terminal 70 amino acids of the adenovirus E4- ORF6 / 7 protein are essential and sufficient for E2F complex formation"
- VIRUS GENES (1990), 4(1), 53-61 CODEN: VIGEET;ISSN: 0920-8569, XP000577713 ROOVERS, DICK J. ET AL: "Physical mapping of two temperature-sensitive adenovirus mutants affected in the DNA polymerase and DNA binding proteins"
- MOLECULAR AND CELLULAR BIOLOGY, 1994, 14, 8166-817 - 0270-7306, XP000568381 SHAN B ET AL: "DEREGULATED EXPRESSION OF E2F-1 INDUCES S-PHASE ENTRY AND LEADS TO APOPTOSIS"
- JOURNAL OF CLINICAL INVESTIGATION, 1994, 93, 1864-1868, XP000568358 FISHMAN GI ET AL: "TETRACYCLINE-REGULATED CARDIAC GENE-EXPRESSION IN-VIVO"
- HUMAN GENE THERAPY, 1995, 6/12 (1575-1586), USA, XP000568394 KROUGLIAK V. ET AL: "Development of cell lines capable of complementing E1, E4, and protein IX defective adenovirus type 5 mutants"
- CANCER GENE THER., 1995, XP000568385 WANG Q ET AL: "A new packaging cell line for propagation of the second generation of the recombinant adeno virus vector"
- Gossen and Bujard: " Tight control of gene expression in mammalian cells by tetracycline-responsive promoters." PNAS, June 1992, vol.89, pages 5547-5551.

## Description

La présente invention concerne de nouveaux vecteurs viraux permettant le transfert et l'expression de gènes d'intérêt dans une cellule ou un organisme hôte, l'expression des gênes viraux étant régulée de manière à être fonctionnelle dans une cellule de complémentation et non-fonctionnelle dans la cellule ou l'organisme hôte. Elle a également pour objet les cellules contenant ces nouveaux vecteurs ainsi qu'une méthode pour préparer des particules virales infectieuses destinées à un usage thérapeutique. L'invention présente un intérêt tout particulier pour des perspectives de thérapie génique, notamment chez l'homme.

La possibilité de traiter les maladies humaines par thérapie génique est passée en quelques années du stade des considérations théoriques à celui des applications cliniques. Le premier protocole appliqué à l'homme a été initié aux Etats Unis en septembre 1990 sur un patient génétiquement immunodéficient en raison d'une mutation affectant le gène codant pour l'Adénine Désaminase (ADA). Le succès relatif de cette première expérimentation a encouragé le développement de nouveaux protocoles de thérapie génique pour diverses maladies génétiques ou acquises (maladies infectieuses et notamment virales comme le SIDA ou cancers). La grande majorité des protocoles décrits jusqu'à présent met en oeuvre des vecteurs viraux pour transférer et exprimer le gène thérapeutique dans les cellules à traiter.

A ce jour, les vecteurs rétroviraux sont parmi les plus utilisés du fait de la simplicité de leur génome. Cependant, outre leur capacité restreinte de clonage, ils présentent deux inconvénients majeurs qui limitent leur utilisation systématique : d'une part, ils infectent majoritairement les cellules en division et d'autre part, du fait de leur intégration au hasard dans le génome de la cellule hôte, le risque de mutagénèse insertionnelle n'est pas négligeable. C'est pourquoi, de nombreuses équipes scientifiques se sont attachées à développer d'autres types de vecteurs, parmi lesquels on peut citer ceux issus des adénovirus, virus associés aux adénovirus (AAV), cytomégalovirus, poxvirus et virus de l'herpès. D'une manière générale, leur organisation et leur cycle d'infection sont largement décrits dans la littérature accessible à l'homme de l'art.

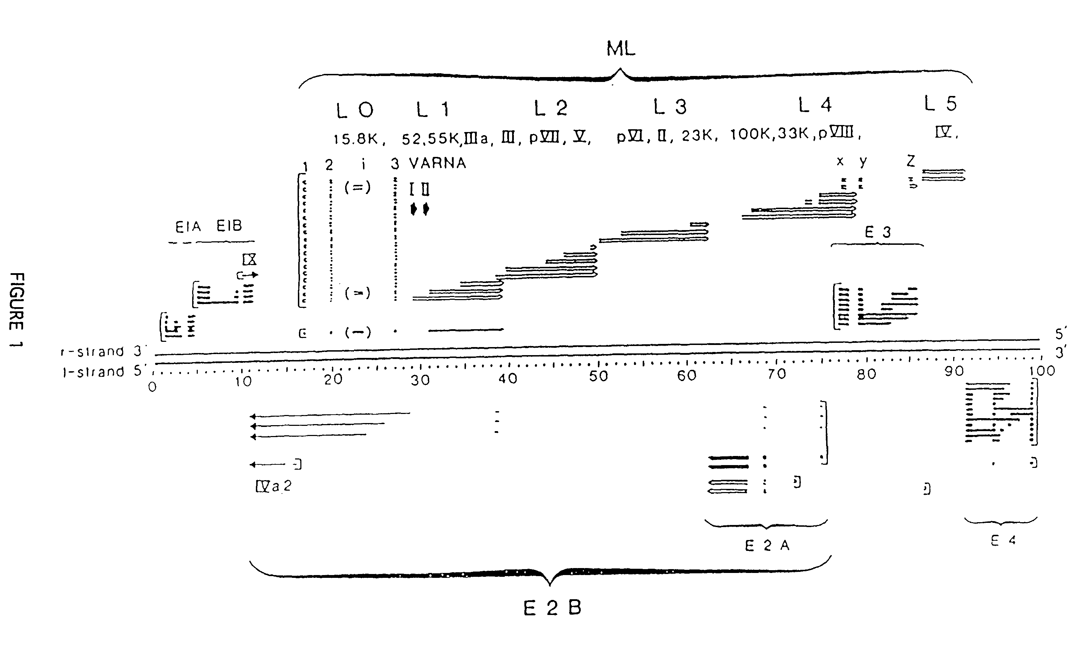

A cet égard, l'emploi des vecteurs adénoviraux est apparu comme une alternative prometteuse. Les adénovirus ont été mis en évidence dans de nombreuses espèces animales, ont un large spectre d'hôtes, sont peu pathogènes et ne présentent pas les inconvénients liés aux rétrovirus puisqu'ils sont non-intégratifs et se repliquent également dans les cellules quiescentes. A titre indicatif, leur génome est constitué d'une molécule d'ADN linéaire et bicaténaire d'environ 36 kb portant plus d'une trentaine de gènes, à la fois des gènes précoces nécessaires à la replication virale et des gènes tardifs de structure (voir Figure 1).

Les gènes précoces sont répartis en 4 régions dispersées dans le génome adénoviral (E1 à E4 ; E pour early signifiant précoce en anglais). Elles comportent 6 unités transcriptionnelles qui possèdent leurs propres promoteurs. Les gènes tardifs (L1 à L5 ; L pour late signifiant tardif en anglais) recouvrent en partie les unités de transcription précoces et sont, pour la plupart, transcrits à partir du promoteur majeur tardif MLP (pour Major Late Promoter en anglais).

A l'heure actuelle, tous les vecteurs adénoviraux utilisés dans les protocoles de thérapie génique sont dépourvus de la majeure partie de la région E1 essentielle à la replication, afin d'éviter leur dissémination dans l'environnement et l'organisme hôte. Certains comportent des délétions supplémentaires, notamment dans la région E3 non essentielle, permettant d'accroître leur capacité de clonage. Les gènes d'intérêt sont introduits dans l'ADN viral à la place de l'une ou l'autre région délétée. La délétion de la région E1 rend le génome viral déficient pour la replication. Cependant, les virus E1⁻ peuvent être propagés dans une lignée cellulaire de complémentation, qui fournit *en trans* les fonctions virales délétées pour générer une particule virale infectieuse. On utilise couramment la lignée 293, établie à partir de cellules de rein embryonnaire humain, qui complémente efficacement la fonction E1 (Graham et al., 1977, J. Gen. Virol. *36*, 59-72). La région E3 est non essentielle et ne nécessite pas d'être complémentée.

Si la faisabilité du transfert de gènes en utilisant ces vecteurs de première génération est maintenant bien établie, la question de leur innocuité reste posée. Outre les aspects de sécurité (risque de générer des RCA c'est à dire des particules compétentes pour la replication), se pose le problème de leur toxicité. En effet, les premiers essais cliniques ont mis en évidence l'induction de réponses inflammatoires liées à l'expression des gènes viraux chez l'hôte.

Des vecteurs adénoviraux de seconde génération ont récemment été proposés dans la littérature. Ils conservent les régions *en cis* nécessaires à la replication du virus dans la cellule infectée (ITRs et séquences d'encapsidation) et comportent des délétions internes importantes visant à supprimer l'essentiel des gènes viraux dont l'expression *in vivo* n'est pas souhaitable. Cependant, ces vecteurs de l'art antérieur présentent certains inconvénients qui limitent leur exploitation à un niveau industriel. Il est, en effet, nécessaire de disposer de nouvelles lignées complémentant l'ensemble des fonctions délétées et permettant la production de particules virales à un titre élevé. Or une telle lignée pour être optimale en terme de capacité de croissance et rendement en particules virales est particulièrement difficile à générer du fait de la cytotoxicité des gènes adénoviraux.

La présente invention permet de remédier à ces inconvénients. On a maintenant construit, d'une part, une nouvelle lignée dérivée de la lignée 293 complémentant les fonctions adénovirales E1 et E2 ou E4, pour l'amplification de vecteurs adénoviraux conventionnels de seconde génération, dans laquelle l'expression des régions E2 ou E4 est dirigée par un promoteur muni en son extrémité 5' de séquences dîtes "opérateurs" de l'opéron bactérien tétracycline désignées ci-après tet O. La synthèse des produits d'expression correspondants ne sera activée qu'en présence d'un inducteur qui peut être produit par le vecteur adénoviral ou par la lignée elle-même. De même, un represseur peut être ajouté dans le milieu de culture lorsque la complémentation n'est plus désirée.

D'autre part, on a maintenant généré de nouveaux vecteurs adénoviraux délétés de la majorité des régions E1 et E3, dans lesquels les unités transcriptionnelles des régions virales restantes (E2, E4 et/ou L1-L5) sont régulables afin de permettre leur expression lorsqu'il s'agit de générer les particules virales infectieuses et de l'inhiber dans la cellule hôte. Dans les exemples qui suivent, la régulation est assurée par les séquences tet O. Leur insertion en 5' de la TATA box génère un promoteur à partir duquel le niveau de transcription basal est minime mais peut être fortement stimulé en présence de l'inducteur cité ci-dessus. Ainsi, la production des protéines virales est activée dans une lignée 293 exprimant l'inducteur, ce qui permettra de constituer les particules virales infectieuses Par contre, elle est considérablement réduite dans la cellule hôte infectée qui ne produit pas naturellement l'inducteur d'origine bactérienne. La régulation des gènes viraux est sans conséquence sur l'expression de la séquence nucléotidique exogène placée sous la dépendance d'un promoteur ne répondant pas à la tétracycline.

A titre d'exemple, WO-A-95 02697 décrit bien des vecteurs adénoviraux qui comprennent des gènes E1 et E2 ou E4 non fonctionnels. Il y est notamment précisé que cette « non fonctionnalité» peut être obtenue par suppression, substitution, délétion ou addition d'une ou plusieurs bases, y compris dans la région promotrice de ces gènes. Cependant, selon un mode de réalisation préférée, il est indiqué que le caractère non fonctionnel est parfaitement stable, ségrégationnel et/ou non-réversible.

Les vecteurs adénoviraux de la présente invention proposent une solution avantageuse aux inconvénients inhérents à l'utilisation des vecteurs de l'art antérieur, puisqu'ils combinent sécurité d'emploi et facilité de production. D'une part, ils peuvent être propagés dans une lignée de complémentation conventionnelle avec un titre élevé compatible avec les besoins industriels et, d'autre part, ils permettent de transférer une séquence nucléotidique exogène *in vivo*, de l'exprimer de manière stable tout en limitant les effets nocifs (réponses inflammatoires chez l'hôte). Ils sont tout particulièrement adaptés à la thérapie génique humaine.

C'est pourquoi la présente invention a pour objet un vecteur viral comprenant une unité d'expression fonctionnelle dans une cellule de complémentation et non fonctionnelle dans une cellule hôte, controlant l'expression d'un ou plusieurs gènes viraux, caracterisé en ce que ladite unité d'expression comprend une ou plusieurs séquences de régulation permettant d'activer l'expression desdits gènes viraux en présence d'un inducteur et d'inhiber ladite expression en présence d'un répresseur.

Au sens de la présente invention, un "vecteur viral" est obtenu à partir d'un virus parental dont le génome a été modifié. Ces modifications peuvent être diverses (délétion, mutation et/ou addition d'un ou plusieurs nucléotides) et localisées dans les régions codantes du génome viral ou en dehors de celles-ci, par exemple dans les régions promotrices. A titre indicatif, certaines séquences virales peuvent être délétées, rendues non fonctionnelles ou substituées par d'autres séquences et, notamment, une séquence nucléotidique exogène dont l'expression est recherchée dans une cellule hôte.

Un vecteur viral selon l'invention, peut dériver de virus très divers, comme les virus de l'herpès, cytomégalovirus, AAV (virus associé à l'adénovirus), poxvirus et notamment virus de la vaccine, fowlpox ou canarypox. De tels vecteurs ainsi que leurs techniques de préparation sont connus de l'homme de l'art.

Cependant, un vecteur particuliérement adapté à la présente invention est un vecteur adénoviral. Il peut dériver d'un adénovirus d'origine humaine, canine, aviaire, bovine, murine, ovine, porcine ou simienne ou encore d'un hybride comprenant des fragments de génome adénoviral de différentes origines. On peut citer plus particulièrement les adénovirus CAV-1 ou CAV-2 d'origine canine, DAV d'origine aviaire ou encore Bad de type 3 d'origine bovine (Zakharchuk et al., 1993, Arch. Virol., *128*, 171-176 ; Spibey et Cavanagh, 1989, J. Gen Virol., *70*, 165-172 ; Jouvenne et al., 1987, Gene, *60*, 21-28 ; Mittal et al., 1995, J. Gen. Virol., *76*, 93-102). Cependant, on préférera un vecteur adénoviral dérivé d'un adénovirus humain, de préférence, de sérotype C et, de manière tout à fait préférée, de type 2 ou 5 (Graham et Prevect, 1991, Methods in Molecular Biology, vol 7, p 109-128 ; Ed : E.J. Murey, The Human Press Inc.).

Un mode de réalisation avantageux de la présente invention consiste en un vecteur défectif pour la replication dans lequel un ou plusieurs gènes viraux nécessaires à la replication sont délétés ou rendus non fonctionnels. Un tel vecteur, incapable de replication autonome, sera propagé dans une cellule de complémentation. Le terme "cellule de complémentation" désigne une cellule capable de fournir *en trans* la ou les fonction(s) pour la(les)quelle(s) un vecteur selon l'invention est défectif. En d'autres termes, elle est capable de produire la ou les protéine(s) nécessaires à la replication et à l'encapsidation dudit vecteur, protéines précoces et/ou tardives, qu'il ne peut lui même produire et qui sont nécessaires à la constitution d'une particule virale infectieuse. A titre illustratif, un vecteur adénoviral préféré selon l'invention étant dépourvu de la majorité de la région E1, on aura recours à une cellule de complémentation telle que la lignée 293 capable de fournir *en trans* l'ensemble des protéines codées par la région E1 que le vecteur ne peut produire. Par "particule virale infectieuse", on entend une particule virale ayant la capacité d'infecter une cellule hôte et d'y faire pénétrer le génome viral.

Selon un mode de mise en oeuvre préférentiel, un vecteur viral selon l'invention est recombinant. Ainsi, il comprendra une séquence nucléotidique exogène placée sous le contrôle des éléments nécessaires à son expression dans une cellule hôte. "Séquence nucléotidique exogène" fait référence à un acide nucléique qui peut être d'une origine quelconque et qui n'est normalement pas présent dans le génome d'un virus parental en usage dans la présente invention ou, s'il est présent, dans un contexte génomique différent. Dans le cadre de l'invention, la séquence nucléotidique exogène peut-être constituée d'un ou plusieurs gènes et, en particulier, de gène(s) d'intérêt thérapeutique.

D'une manière générale, la séquence nucléotidique exogène peut coder pour un ARN anti-sens et/ou un ARNm qui sera ensuite traduit en protéine d'intérêt. Elle peut être de type génomique, de type ADN complémentaire (ADNc) ou de type mixte (minigène, dans lequel au moins un intron est délété). Elle peut coder pour une protéine mature ou un précurseur d'une protéine mature, notamment un précurseur destiné à être secrété et comprenant un peptide signal. Par ailleurs, il peut s'agir de tout ou partie d'une protéine telle que trouvée dans la nature (protéine native ou tronquée) ou également d'une protéine chimérique provenant de la fusion de séquences d'origines diverses ou encore mutée présentant des propriétés biologiques améliorées et/ou modifiées. De telles protéines peuvent être obtenues par les techniques conventionnelles de biologie moléculaire.

Dans le cadre de la présente invention, il peut être avantageux d'utiliser les gènes codant pour les polypeptides suivants:
- cytokines ou lymphokines (interférons α, β et γ, interleukines et notamment l'IL-2, l'IL-6, l'IL-10 ou l'IL-12, facteurs nécrosant des tumeurs (TNF), facteurs stimulateurs de colonies (GM-CSF, C-CSF, M-CSF...) ;
- récepteurs cellulaires ou nucléaires (récepteurs reconnus par des organismes pathogènes (virus, bactéries, ou parasites) et, de préférence, par le virus VIH (Virus de l'Immunodéficience Humain) ou leurs ligands ;
- protéines impliquées dans une maladie génétique (facteur VII, facteur VIII, facteur IX, dystrophine ou minidystrophine, insuline, protéine CFTR (Cystic Fibrosis Transmembrane Conductance Regulator), hormones de croissance (HGF) ;
- enzymes (uréase, rénine, thrombine) ;
- inhibiteurs d'enzymes (α1-antitrypsine, antithrombine III, inhibiteurs de protéases virales...);
- polypeptides à effet anti-tumoral capables d'inhiber au moins partiellement l'initiation ou la progression de tumeurs ou cancers (ARN anti-sens, anticorps, inhibiteurs agissant au niveau de la division cellulaire ou des signaux de transduction, produits d'expression des gènes suppresseurs de tumeurs, par exemple p53 ou Rb, protéines stimulant le système immunitaire....);
- protéines du complexe majeur d'histocompatibilité des classes I ou II ou protéines régulatrices agissant sur l'expression des gènes correspondants ;
- polypeptides capables d'inhiber une infection virale, bactérienne ou parasitaire et/ou son développement (polypeptides antigéniques ayant des propriétés immunogènes, épitopes antigéniques, anticorps, variants trans-dominants susceptibles d'inhiber l'action d'une protéine native par compétition...) ;
- toxines ( thymidine kinase de virus simplex de l'herpès 1 (TK-HSV-1), ricine, toxine cholérique, diphtérique) ou immunotoxines ; et
- marqueurs (β-galactosidase, luciférase....).

Il est à signaler que cette liste n'est pas limitative et que d'autres gènes peuvent également être employés.

Par ailleurs, une séquence nucléotidique exogène en usage dans la présente invention peut, en outre, comprendre un gène de sélection permettant de sélectionner ou identifier les cellules transfectées. On peut citer les gènes *néo* (codant pour la néomycine phosphotransférase) conférant une résistance à l'antibiotique G418, *dhfr* (Dihydrofolate Réductase), CAT (Chloramphenicol Acetyl transférase), *pac* (Puromycine Acétyl-Transferase) ou encore *gpt* (Xanthine Guanine Phosphoribosyl Transferase). D'une manière générale, les gènes de sélection sont connus de l'homme de l'art.

Par éléments nécessaires à l'expression d'une séquence nucléotidique exogène dans une cellule hôte, on entend l'ensemble des éléments permettant sa transcription en ARN (ARN anti-sens ou ARNm) et la traduction d'un ARNm en protéine. Parmi ceux-ci, le promoteur revêt une importance particulière. Il peut être isolé d'un gène quelconque d'origine eucaryote ou même virale et peut être constitutif ou régulable. Alternativement, il peut s'agir du promoteur naturel du gène en question. On préférera employer un promoteur différent de celui indu dans l'unité d'expression des gènes viraux (définie ci-après). Par ailleurs, il peut être modifié de manière à améliorer l'activité promotrice, supprimer une région inhibitrice de la transcription, rendre un promoteur constitutif régulable ou vice versa, introduire un site de restriction..... On peut mentionner, à titre d'exemples, les promoteurs des gènes TK-HSV-1, PGK (Phospho Glycerate kinase) murin ou humain, α1-antitrypsine (foie-spécifique), immunoglobulines (lymphocyte-spécifique), le promoteur précoce du virus SV40 (Simian Virus 40), un LTR rétroviral, ou encore le promoteur adénoviral MLP, notamment de l'adénovirus humain de type 2.

Bien entendu, une séquence nucléotidique exogène en usage dans la présente invention peut en outre comprendre des éléments additionnels nécessaires à l'expression (séquence intronique, séquence signal, séquence de localisation nucléaire, séquence terminatrice de la transcription, site d'initiation de la traduction de type IRES ou autre....) ou encore à sa maintenance dans la cellule hôte. De tels éléments sont connus de l'homme de l'art.

Comme indiqué précédemment, un vecteur viral selon la présente invention comprend une unité d'expression comportant un ou plusieurs gènes viraux et ayant la caractéristique intéressante d'être fonctionnelle dans une cellule de complémentation et non fonctionnelle dans une cellule hôte. Par "fonctionnelle", on entend une expression des gênes viraux en quantité suffisante et pendant un temps suffisamment long, pour permettre la constitution d'une particule virale infectieuse. Par "non fonctionnelle", on entend une expression des gènes viraux réduite (de préférence d'au moins un facteur 10, voire même nulle) par rapport à leur niveau d'expression dans le virus parental. Le caractère fonctionnel se manifeste par la production des produits des gènes viraux inclus dans l'unité d'expression, ceux-ci pouvant être mis en évidence par les techniques classiques de biologie moléculaire, d'immunologie, de biochimie ou d'enzymologie. Le caractère non fonctionnel se manifeste par l'absence de production ou encore, par la production des produits viraux à un niveau atténué.

Ainsi, ladite unité d'expression comprend une ou plusieurs séquence(s) de régulation hétérologue(s) non présente(s) dans l'ADN viral parental. On peut avoir recours à des séquences répondant à un régulateur de type répresseur agissant de manière négative sur l'expression ou, de préférence, à un régulateur de type inducteur exerçant une action positive. Une séquence de régulation mise en oeuvre dans le cadre de la présente invention peut être d'une origine quelconque, virale, procaryote ou encore eucaryote. D'une manière générale, les séquences de régulation sont décrites dans la littérature accessible à l'homme de l'art. Il peut également s'agir d'un homologue dont la séquence est modifiée par rapport à la séquence native mais exerçant une fonction de régulation similaire ou améliorée. Ces modifications peuvent résulter de l'addition, de la délétion et/ou de la substitution d'un ou plusieurs nucléotides.

Dans le cadre de la présente invention, une séquence de régulation est susceptible de moduler l'expression des gènes viraux à différents niveaux : transcription, élongation, transport, stabilité des ARNm ou encore traduction. Elle peut être présente à des endroits divers de ladite unité d'expression, par exemple, dans le promoteur surtout lorsque son effet se situe au niveau de la transcription (de préférence en amont de la TATA box jusqu'à plusieurs centaines de paires de bases de celle-ci) ou dans les séquences transcrites (et si possible non codantes) lorsque son action s'exerce à une étape ultérieure de la transcription. On peut mettre en oeuvre de 1 à 25 séquences de régulation, avantageusement, de 1 à 20, de préférence, de 1 à 10 et, de manière tout à fait préférée, de 1 à 7.

Au sens de la présente invention, le terme "inducteur" désigne une molécule qui a la capacité d'initier ou d'activer l'expression des gènes viraux placés sous la dépendance d'une séquence de régulation, soit directement par liaison à ladite séquence de régulation soit indirectement par l'intermédiaire d'autres facteurs cellulaires ou viraux. Il peut également empêcher l'action d'un répresseur. A *contrario*, un "répresseur", a la capacité d'inhiber ou de bloquer l'expression des gènes viraux placés sous la dépendance d'une séquence de régulation sur laquelle il agit et ceci soit directement ou indirectement.

On peut illuster ces définitions par l'exemple de l'opéron lactose (*lac*). Le gène *lac*I code pour un répresseur qui se fixe à une courte séquence de régulation dîte "opérateur" empêchant ainsi la transcription des gènes structuraux codant pour les enzymes de la chaîne métabolique du lactose. En présence de l'inducteur, celui-ci se fixe au répresseur et le transforme en forme inactive qui ne peut plus se lier à l'opérateur permettant ainsi à la transcription de se dérouler.

Il est également envisageable d'utiliser des portions ou des analogues de ces régulateurs afin d'améliorer leur efficacité ou modifier leur spécificité (par exemple l'anhydro tétracycline dix fois plus efficace que la tétracycline pour inhiber la transcription à partir d'un promoteur comprenant les séquences de régulation dérivant de l'opéron tétracycline ou le transactivateur reverse décrit récemment par Gossen et al., 1995, Science, *268*, 1766-1769). Par ailleurs, un régulateur en usage dans la présente invention peut être une protéine hybride provenant de la fusion de polypeptides d'origines diverses. Une combinaison préférée consiste en un polypeptide capable de reconnaître ou lier une séquence de régulation en usage dans la présente invention (par exemple dérivé du répresseur tetracycline (tet R) ou estrogène ER) et un polypeptide capable d'activer l'expression (par exemple le domaine d'activation des protéines Ga14 ou VP16 capable d'interagir avec les facteurs de transcription).

Le tableau 1 suivant cite quelques unes des séquences de régulation et régulateurs utilisables dans le cadre de la présente invention :

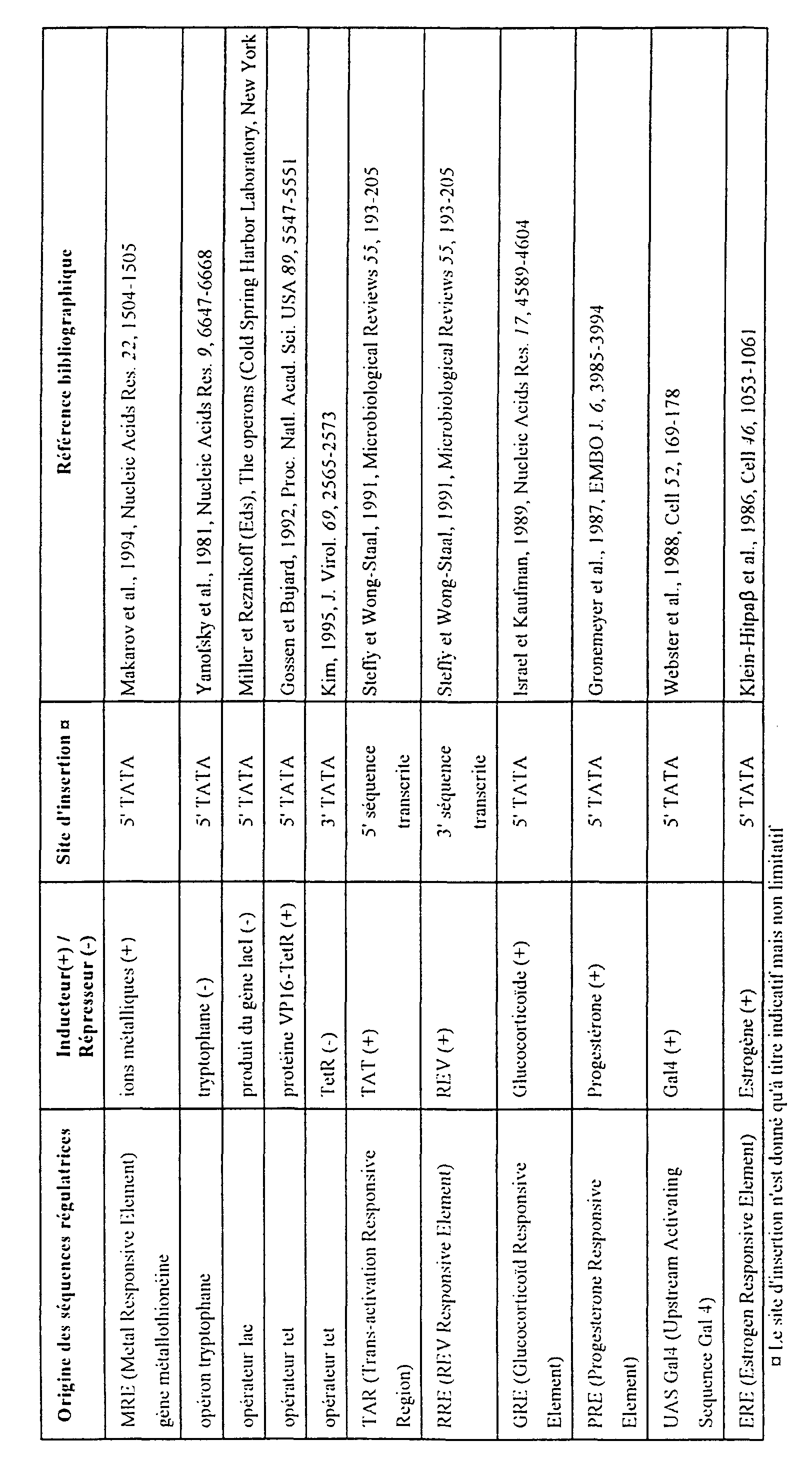

Selon un mode de réalisation particulier de la présente invention, on utilise des séquences de régulation dérivant de l'opéron bactérien tétracycline désignées dans la littérature "opérateur" (tet O). D'une manière générale, l'opéron de résistance à la tétracycline est codé par le transposon Tn10 (Hillen et al., 1984, J. Mol. Biol. *172*, 185-201). La régulation est assurée par une courte séquence nucléotidique dite "opérateur" (tet O) qui constitue un site de fixation de divers régulateurs. Ainsi, la fixation du répresseur tétacycline (tet R) ou de l'antibiotique tétracycline diminue considérablement le niveau de transcription. Au contraire, un effet d'activation est obtenu en mettant en oeuvre une protéine, désignée dans la littérature "trans-activateur tétracycline (tTA)" qui résulte de la fusion entre le tet R et les 130 acides aminés C-terminaux du domaine d'activation de la protéine VP16 du virus simplex de l'herpès. Gossen et Boujard (1992, Proc. Natl. Acad. Sci. USA *89*, 5547-5551) ont récemment montré que ce système de régulation est fonctionnel dans les cellules eucaryotes. L'expression d'un gène reporter placé sous le contrôle de plusieurs copies de tet O en amont de séquences de base de la transcription (TATA box, site d'initiation de la transcription..) est détectable par co-expression du tTA et inhibée par ajout de tétracycline. Quant au groupe de Kim (1995, J. Virol. *69*, 2565-2573), il utilise les séquences tet O positionnées en aval de la TATA box d'un promoteur et, dans ce cas, la transcription est inhibée par action de tetR.

Dans le cadre de la présente invention, on préfère tout particulièrement la combinaison "tet O - promoteur minimal" (dans le sens 5' vers 3') donnant lieu à un promoteur dont le niveau de base de la transcription est naturellement très faible mais activable par l'inducteur tTA et répressible par la tétracycline. Cependant, il est également possible d'utiliser un promoteur dans lequel les séquences tet O sont placées en 3' de la "TATA box" ou encore de part et d'autre de celle-ci, répressible par un répresseur comprenant les séquences du tet R reconnaissant tet O.

S'agissant de la variante préférée, un vecteur adénoviral selon l'invention est, de préférence, constitué par le génome d'un adénovirus dépourvu de tout ou partie de la région E1 et, de manière alternative, de tout ou partie de la région E3. Avantageusement, on préfère conserver une partie de la région E3 et, notamment, la partie correspondant au gène codant pour la gp19k (Gooding and Wold, 1990, Critical Reviews of Immunology *10*, 53-71), ladite partie n'étant pas incluse dans une unité d'expression telle que définie ci-avant mais placée sous le contrôle d'un promoteur homologue (E3) ou hétérologue conventionnel. Il va de soi que l'on peut procéder à d'autres modifications, du génome viral notamment dans la région E4 ou E2. Il peut être intéressant d'introduire des délétions supplémentaires ou des mutations. Pour illuster ce point, on peut citer la mutation thermosensible affectant le gène DBP (pour DNA Binding Protein en anglais) de la région E2A (Ensinger and Ginsberg, 1972, J. Virol. *10*, 328-339).

Selon un mode de réalisation préférentiel, un vecteur adénoviral selon l'invention comprend une unité d'expression comportant un ou plusieurs gènes viraux des régions E2, E4 ou L1-L5. Une variante intéressante consiste à ne retenir de la région E4 que les séquences codant pour les ORFs 3, 6 et/ou 7 (ces délétions limitées de la région E4 ne nécessitent pas de complémentation de la fonction E4 ; Ketner et al., 1989, Nucleic Acids Res. *17*, 3037-3048).

Il peut également être intéressant de disposer d'un vecteur adénoviral comprenant plusieurs unités d'expression, toutes les combinaisons étant envisageables (E2 et E4, E2 et L1-L5, E4 et L1-L5 ou E2, E4 et L1-L5). On choisira alors des séquences de régulation agissant d'une même manière et, de préférence, positivement (par exemple TAR, RRE, tet O...). Pour des raisons de simplicité de mise en oeuvre, on préfère le cas où les unités portent des séquences de régulation identiques permettant de propager le vecteur adénoviral dans une lignée de complémentation comprenant un seul inducteur.

L'invention a également trait à une particule virale infectieuse ainsi qu'à une cellule hôte eucaryote comprenant un vecteur viral selon l'invention. Ladite cellule hôte est avantageusement une cellule de mammifère et, de préférence, une cellule frumaine et peut comprendre ledit vecteur sous forme intégrée dans le génome ou non intégrée (épisome). Il peut s'agir d'une cellule primaire ou tumorale d'une origine hématopoïétique (cellule souche totipotente, leucocyte, lymphocyte, monocyte ou macrophage...), musculaire, pulmonaire, hépatique, épithéliale ou fibroblaste.

Par ailleurs, les inventeurs ont mis au point une cellule de complémentation caractérisée en ce qu'elle comprend un inducteur et/ou un répresseur (réulateur). Selon les besoins, celui-ci peut être ajouté dans le milieu de culture ou produit par la cellule elle-même d'une manière stable ou transitoire. Cette cellule de complémentation peut être modifiée par introduction d'un fragment d'ADN codant pour ledit régulateur. Tous les moyens standards pour introduire un acide nucléique (vecteur synthétique, viral, plasmide, ADN nu...) dans une cellule peuvent être utilisés, comme par exemple la transfection, l'électroporation, la microinjection, la lipofection, l'adsorption et la fusion de protoplastes. Dans le contexte de l'invention, il peut être intéressant de générer une cellule de complémentation qui ne produit qu'un seul régulateur et, notamment, un inducteur. Toutefois, il peut aussi être avantageux de disposer de cellules produisant plusieurs inducteurs.

Selon un mode de réalisation avantageux, une telle cellule de complémentation est capable de complémenter *en trans* un vecteur viral selon l'invention et, en particulier, un vecteur adénoviral. A cet égard, on choisira avantageusement une cellule de complémentation pour la fonction E1 et/ou E4. C'est pourquoi, on peut considérer que ce type de cellule est destiné à la complémentation d'un vecteur adénoviral de seconde génération défectif pour la fonction E1 et une autre fonction adénovirale (tardive ou précoce) Une telle cellule comprend tout ou partie de la région E1 d'un adénovirus dont l'expression est contrôlée par un promoteur quelconque et tout ou partie d'une région d'un adénovirus autre que la région El placée sous le contrôle d'un promoteur muni de séquences de régulation, par exemple en son extrémité 5' d'au moins une et, de préférence une à 20 séquence(s) tet O activable(s) par le trans-activateur tTA. Une variante intéressante consiste en un promoteur minimal dérivé du virus CMV (Cytomégalo virus) en amont duquel se situent 7 séquences tet O dans une orientation tête à queue. L'inducteur peut être introduit dans la cellule de complémentation telle que décrite ci-dessus de manière préalable, concomitante ou postérieure aux séquences adénovirales placées sous le contrôle des séquences tet O ou comme indiqué plus haut il peut être ajouté au milieu de culture. On peut également envisager d'exprimer l'inducteur (par exemple le tTA) dans la cellule de complémentation et d'ajouter le represseur (par exemple la tétracycline) dans le milieu de culture lorsque l'expression des gènes adénoviraux n'est plus désirée.

A titre d'exemples préférés, la région adénovirale autre que la région E1 est constituée par :
(i) tout ou partie de la région E4 et notamment les séquences codant pour les cadres de lecture ouverts 6 et 7 (ORFs 6/7) de cette dernière, ou encore
(ii) tout ou partie de la région E2 et notamment les séquences codant pour la protéine DBP (DNA Binding Protein) ou un mutant thermosensible de cette dernière.

Bien entendu, une telle cellule de complémentation peut en outre comprendre une troisième région adénovirale dont l'expression est placée sous le contrôle des éléments adéquats. A cet égard, une cellule préférée est destinée à la complémentation d'un vecteur adénoviral défectif pour l'ensemble des fonctions précoces essentielles à la replication et comprend tout ou partie des régions E1, E2 et E4, l'une ou l'autre de ces deux dernières régions ou les deux étant placée(s) sous le contrôle d'un promoteur muni de séquence(s) de régulation telles que définies ci-dessus.

Un des avantages de cette cellule de complémentation est qu'elle permet la production à haut titre de particules virales infectieuses à partir d'un vecteur viral conventionnel ou selon l'invention. Le titre viral de la préparation finale (après purification) est avantageusement supérieur à 5x10⁸ pfu/ml, de préférence supérieur à 1x10⁹ pfu/ml, de manière tout à fait préférée, supérieur à 5x10⁹ pfu/ml et, de manière encore plus préférée, supérieur à 5x10¹⁰ pfu/ml. Par pfu, on entend une particule capable de former une plage par infection de cellules permissives. Les techniques pour évaluer le nombre de pfu sont conventionnelles et connues de l'homme de l'art. On peut citer la technique en agar (Graham et Prevec, 1991, *supra*). Généralement, le titre viral en pfu/ml est égal ou inférieur au nombre réel de particules virales infectieuses susceptibles de remplir leur fonction de transfert de gênes. En effet, un certain pourcentage est incapable de se propager efficacement et donc générer des plages de lyse sur cellules permissives. Le titre en particules virales infectieuses produit par la susdite cellule de complémentation est avantageusement supérieur à 5x10⁹ ifu/ml, de préférence supérieur à 1x10¹⁰ ifu/ml, de manière tout à fait préférée, supérieur à 5x10¹⁰ ifu/m, et de manière encore plus préférée, supérieur à 5x10¹¹ ifu/ml Par ifu, on entend une particule infectieuse capable d'infecter une cellule cible non permissive et de transférer son génome et permettre l'expression des gènes portés par ce dernier. Le nombre d'ifu est estimé par le nombre de cellules cibles exprimant le gène d'intérêt ou un gène viral. L'homme de l'art connaît les techniques à mettre en oeuvre pour détecter leur expression : par immunofluorescence, Western blot ou encore coloration..... Par exemple, lorsque le gène d'intérêt est constitué par le gène LacZ, la protéine β-galactosidase peut être visualisée par coloration au X-Gal (5-bromo-4-chloro-3-indolyl-β-D-galactopyranoside) et on dénombre le nombre de cellules bleues. Lorsqu'on emploie le gène thérapeutique CFTR, le produit d'expression peut être visualisé par Western blot. On peut également rechercher les cellules exprimant les protéines adénovirales DBP penton ou fibre à l'aide d'anticorps spécifiques.

Une cellule de complémentation telle que définie ci-dessus peut être générée à partir de diverses lignées cellulaires par transfection de portions appropriées du génome adénoviral et d'un fragment d'ADN codant pour un régulateur. Parmi les lignées envisageables, on peut citer les lignées de rein Véro (singe), BHK (hamster), MDCK (chien) MBDK (bovin), la lignée CHO (hamster) ou encore les lignées humaines (HeLa, A549, MRCS, W138...) disponibles dans les collections telles que l'ATCC (Rockville, USA). Cependant, une cellule particulièrement adaptée est la lignée 293. On peut également envisager l'utilisation de lignées primaires telles que des cellules primaires de rétine humaine.

Une particule virale infectieuse selon l'invention peut être préparée selon toute technique conventionnelle dans le domaine de l'art (Graham et Prevect, 1991, *supra*), par exemple, par co-transfection d'un vecteur et d'un fragment adénoviral dans une cellule appropriée ou encore par le moyen d'un virus auxiliaire fournissant *en trans* les fonctions virales non fonctionnelles. Il est également envisageable de générer le vecteur viral *in vitro* dans *Escherichia coli (E. coli)* par ligation ou encore recombinaison homologue (voir par exemple la demande française N° 2727689 (94 14470)).

L'invention concerne également un procédé de préparation d'une particule virale infectieuse comprenant un vecteur viral selon l'invention, selon lequel :
(i) on introduit ledit vecteur viral dans une cellule de complémentation capable de complémenter *en trans* ledit vecteur, de manière à obtenir une cellule de complémentation transfectée,
(ii) on cultive ladite cellule de complémentation transfectée dans des conditions appropriées pour permettre l'expression des gènes viraux et la production de ladite particule virale infectieuse, et
(iii) on récupère ladite particule virale infectieuse dans la culture cellulaire.

Bien entendu, la particule virale infectieuse peut être récupérée du surnageant de culture niais également des cellules. Selon un mode de réalisation avantageux, on met en oeuvre un vecteur adénoviral et une cellule de complémentation telle que précédemment décrite. Selon une autre variante, on peut avoir recours à une cellule de complémentation conventionnelle. Il peut être nécessaire d'ajouter un inducteur au milieu de culture dans le cas où l'unité d'expression comprend des séquences de régulation activables. La quantité à utiliser dépend de la nature même de l'inducteur. L'homme du métier saura bien évidemment adapter la concentration optimale en fonction des données spécifiques.

L'invention a également trait à un procédé de préparation d'une particule virale infectieuse comprenant un vecteur viral conventionnel mettant en oeuvre la susdite cellule de complémentation. A titre d'exemple, l'introduction d'un vecteur viral défectif pour les fonctions E1 et E4 (E1- E4-) dans une cellule 293 exprimant (i) les ORFs 6/7 de la région E4 sous le contrôle d'un promoteur minimal muni en son extrémité 5' de 7 séquences tet O et (ii) le trans-activateur tTA dirigé par le même promoteur permettra de générer des panicules virales déficientes pour la replication mais infectieuses à l'égard d'une cellule hôte.

L'invention a également pour objet une composition pharmaceutique comprenant à titre d'agent thérapeutique ou prophylactique, un vecteur viral, une particule virale infectieuse, une cellule de complémentation ou une cellule hôte eucaryote selon l'invention en association avec un support acceptable d'un point de vue pharmaceutique. La composition selon l'invention est en particulier, destinée au traitement préventif ou curatif de maladies telles que :
- des maladies génétiques (hémophilie, mucoviscidose, diabète ou myopathie, celle de Duchène et de Becker..),
- des cancers, comme ceux induits par des oncogénes ou des virus,
- des maladies virales, comme l'hépatite B ou C et le SIDA (syndrome de l'immunodéficience acquise résultant de l'infection par le VIH), et
- des maladies virales récurrentes, comme les infections virales provoquées par le virus de l'herpès.

Une composition pharmaceutique selon l'invention peut être fabriquée de manière conventionnelle. En particulier, on associe une quantité thérapeutiquement efficace d'un agent thérapeutique ou prophylactique à un support tel qu'un diluant. Une composition selon l'invention peut être administrée par voie locale, systémique ou par aérosol, en particulier par voie intragastrique, sous-cutanée, intracardiaque, intramusculaire, intraveineuse, intrapéritonéale, intratumorale intrapulmonaire, intranasale ou intratrachéale. L'administration peut avoir lieu en dose unique ou répétée une ou plusieurs fois après un certain délai d'intervalle. La voie d'administration et le dosage appropriés varient en fonction de divers paramètres, par exemple, de l'individu ou de la maladie à traiter ou encore du ou des gène(s) d'intérêt à transférer. En particulier, les particules virales selon l'invention peuvent être formulées sous forme de doses comprises entre 10⁴ et 10¹⁴ ufp (unités formant des plages), avantageusement 10⁵ et 10¹³ ufp et, de préférence, 10⁶ et 10¹¹ ufp. La formulation peut également inclure un adjuvant ou un excipient acceptable d'un point de vue pharmaceutique.

Enfin, la présente invention est relative à l'usage thérapeutique ou prophylactique d'un vecteur viral, d'une particule virale infectieuse, d'une cellule de complémentation ou d'une cellule hôte eucaryote selon l'invention pour la préparation d'un médicament destiné au traitement du corps humain ou animal et, préférentiellement, par thérapie génique. Selon une première possibilité, le médicament peut être administré directement *in vivo* (par exemple par injection intraveineuse, dans une tumeur accessible, dans les poumons par aérosol...). On peut également adopter l'approche *ex vivo* qui consiste à prélever des cellules du patient (cellules souches de la moëlle osseuse, lymphocytes du sang périphérique, cellules musculaires...), de les transfecter ou infecter *in vitro* selon les techniques de l'art et de les réadminister au patient. Dans le cas où l'unité d'expression comprend des séquences de régulation répondant à un répresseur, on peut envisager l'administration du répresseur pour bloquer ou limiter l'expression des gènes viraux (administration préalable, concommitante ou postérieure du répresseur ou co-expression via les vecteurs conventionnels).

L'invention s'étend également à une méthode de traitement selon laquelle on administre une quantité thérapeutiquement efficace d'un vecteur viral, d'une particule virale, d'une cellule hôte eucaryote ou d'une cellule de complémentation selon l'invention à un patient ayant besoin d'un tel traitement.

La présente invention est plus complètement décrite en référence aux Figures suivantes et à l'aide des exemples suivants.

La Figure 1 est une représentation schématique du génome de l'adénovirus humain de type 5 (représenté en unités arbitraires de 0 à 100), indiquant l'emplacement des différents gènes.

La Figure 2 est une représentation schématique du vecteur pTG4673, permettant l'expression constitutive du tTA dirigée par le promoteur CMV (pCMV) et du gène de sélection *pac* (résistance à la puromycine) sous le contrôle du promoteur SV40.

La Figure 3 est une représentation schématique du vecteur adénoviral pTG4696 comportant une cassette d'expression des ORFs 6 et 7 de la région E4 activable par le tTA.

La Figure 4 est une représentation schématique du vecteur pTG8343 comportant une partie de l'extrémité 5' du génome de l'adénovirus 5.

La Figure 5 est une représentation schématique du vecteur pTG4662, vecteur adénoviral recombinant de première génération délété de l'essentiel des régions E1 et E3. La cassette recombinante est constituée par le promoteur adénoviral MLP (Ad2) suivi du gène bactérien LacZ codant pour la β-galactosidase et du signal de polyadénylation (pA) du virus SV40.

La Figure 6 est une représentation schématique du vecteur pTG4682, vecteur adénoviral délété de l'essentiel des régions E1 et E3 et comportant une cassette d'expression constitutive du tTA.

La Figure 7 est une représentation schématique du vecteur pTG4683, vecteur adénoviral délété de l'essentiel des régions E1, E3 et E4 et comportant une cassette d'expression constitutive du tTA.

La Figure 8 est une représentation schématique du vecteur pTG4675 permettant l'expression du tTA de manière inductible (sous le contrôle du promoteur minimal CMV (-53 à +1) précédé de 7 copies de séquences tet O, indiqué sur la figure tet O - CMVi).

La Figure 9 est une représentation schématique du vecteur pTG4684, vecteur adénoviral délété de l'essentiel des régions E1 et E3 et comportant une cassette d'expression inductible du tTA.

La Figure 10 est une représentation schématique du vecteur pTG4685, vecteur adénoviral délété de l'essentiel des régions E1, E3 et E4 et comportant une cassette d'expression inductible du tTA.

La Figure 11 est une représentation schématique du vecteur pTG5606 permettant l'expression des ORFs 6/7 de la région adénovirale E4 et du tTA sous le contrôle du promoteur minimal CMV précédé de 7 copies de séquences tet O et du gène de sélection *pac* conférant la résistance à la puromycine.

La Figure 12 est une analyse par Western blot de l'expression de la protéine DBP dans les cellules 293 transfectées de manière transitoire par le vecteur pTG9579 et cultivées en présence (+) et en absence (-) de tétracycline.

### EXEMPLES

Les exemples suivants n'illustrent qu'un mode de réalisation de la présente invention.

Les constructions décrites ci-dessous sont réalisées selon les techniques générales de génie génétique et de clonage moléculaire, détaillées dans Maniatis et al., (1989, Laboratory Manual, Cold Spring Harbor, Laboratory Press, Cold Spring Harbor, NY) ou selon les recommandations du fabricant lorsqu'on utilise un kit commercial. Les étapes de clonage mettant en oeuvre des plasmides bactériens sont réalisées dans la souche *E. coli* 5K (Hubacek et Glover, 1970, J. Mol. Biol. *50*, 111-127) ou BJ 5183 (Hanahan, 1983, J. Mol. Biol. *166*, 557-580). On utilise préférentiellement cette dernière souche pour les étapes de recombinaison homologue. Les techniques d'amplification par PCR sont connues de l'homme de l'art (voir par exemple PCR Protocols-A guide to methods and applications, 1990, edité par Innis, Gelfand, Sninsky et White, Academic Press Inc). S'agissant de la réparation des sites de restriction, la technique employée consiste en un remplissage des extrémités 5' protubérantes à l'aide du grand fragment de l'ADN polymérase I d'*E. coli* (Klenow).

En ce qui concerne la biologie cellulaire, les cellules sont transfectées selon les techniques standards bien connues de l'homme du métier. On peut citer la technique au phosphate de calcium (Maniatis et al., *supra*), mais tout autre protocole peut également être employé, tel que la technique au DEAE dextran, l'électroporation, les méthodes basées sur les chocs osmotiques, la microinjection ou les méthodes basées sur l'emploi de liposomes. Quant aux conditions de culture, elles sont classiques.

Dans les exemples qui suivent, on a recours aux lignées cellulaires suivantes :
Lignée 293 dérivée de rein embryonnaire humain (Graham et al, 1977, *supra*) qui résulte de l'intégration dans ses chromosomes de l'extrémité 5' du génome de l'Ad5 (ITR5', séquence d'encapsidation et région E1) (disponible à l'ATCC sous la référence CRL1573).
Lignée TG1653 (décrite dans la demande internationale WO94/28152, exemple 8) qui dérive de la lignée 293 transformée de manière stable par le plasmide pTG1653 portant la région E4 de l'Ad5 (nt 32800 à 35826) et la cassette d'expression du gène de sélection *pac*.

Il est entendu que d'autres lignées cellulaires peuvent être utilisées.

Par ailleurs, les fragments de génome adénoviral employés dans les différentes constructions décrites ci-après, sont indiqués précisément selon leur position dans la séquence nucléotidique du génome de l'Ad5 telle que divulguée dans la banque de données Genebank sous la référence M73260.

### EXEMPLE 1 : lignée de complémentation exprimant un inducteur capable d'activer l'expression des gènes adénoviraux

Cet exemple décrit la constitution (1) d'une lignée de complémentation dérivant de la lignée 293 et capable d'exprimer de manière constitutive la protéine trans-activatrice tTA et (2) d'un vecteur adénoviral dans lequel certains gènes viraux de la région E4 ont été placés sous le contrôle des séquences tet O répondant au tTA. La transfection du vecteur dans la lignée est suivie de la formation de particules virales puisque les fonctions E1 et E4 sont trans-complémentées par l'apport des produits d'expression de la région adénovirale E1 et du tTA. Dans les cellules hôtes infectées qui ne produisent pas naturellement la protéine chimère tTA, l'expression de E4 et, par voie de conséquence, des protéines tardives (expression dépendante de E4) sera considérablement réduite, limitant ainsi les problèmes d'immunité cellulaire et d'inflammation.

### 1. Constitution d'une lignée de complémentation exprimant le trans-activateur tTA

On construit tout d'abord le vecteur pTG6529 permettant la selection des cellules transformées par la puromycine. Le vecteur résulte du clonage dans un p polyIII-I* (Lathe et al., 1987, Gene *57*, 193-201) d'une cassette d'expression composée du promoteur SV40, du gène *pac* suivi du signal polyA du virus SV40. Une telle construction est à la portée de l'homme de l'art à partir des séquences correspondantes décrites dans la littérature (Morgenstern and Land, 1990, Nucleic Acid Res. 18, 3587-3596 ; Genebank référence J02400).

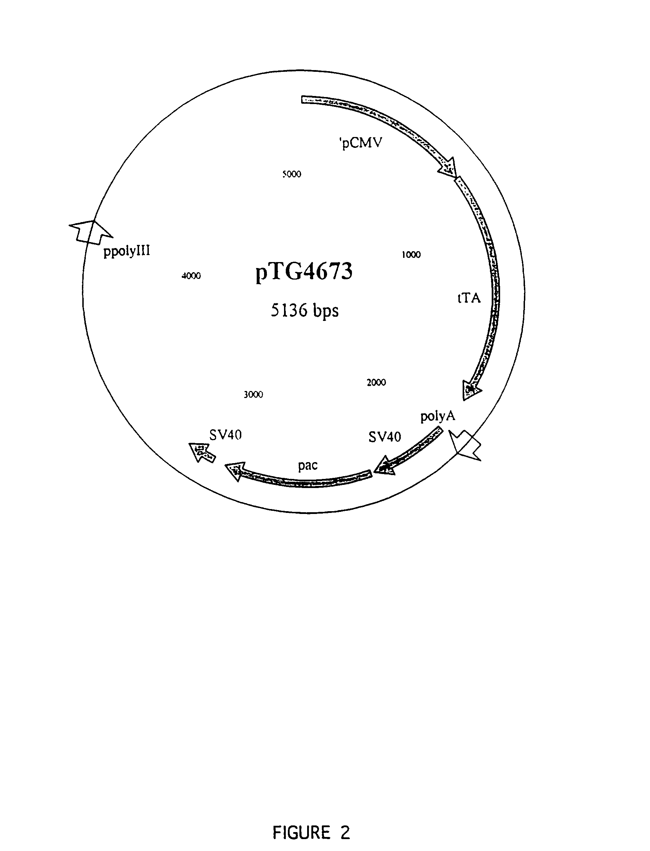

En parallèle, le vecteur pUHD15-1 (Gossen et Bujard, 1992, *supra*) est digéré par les enzymes *Ssp*I et *Hpa*I. Le fragment comportant les séquences codant pour le tTA précédées du promoteur CMV, est cloné entre les mêmes sites du pTG6529 pour donner pTG4673 (Figure 2).

Le vecteur pTG4673 est transfecté de manière conventionnelle dans les cellules 293. Bien entendu, on aurait pu également transfecter un vecteur d'expression du tTA et un vecteur de sélection (par exemple puHD15-1 et pTG6529). Après transfection, les cellules sont cultivées en milieu sélectif (1 µg/ml de puromycine). 80 clones résistants ont été isolés et, parmi eux, 35 testés pour leur capacité à transactiver un gène marqueur dont l'expression est contrôlée par les séquences tet O. Pour des raisons de simplicité de mise en oeuvre et de sensibilité de dosage, le choix s'est porté sur le gène luciférase.

On utilise le vecteur reporteur pUHC13-3 (Gossen et Bujard, 1992, *supra*) dans lequel les séquences codant pour la luciférase sont placées sous le contrôle du promoteur minimal CMV précédé par 7 copies de tet O. Le test est effectué par transfection transitoire dans les clones à tester. Deux jours plus tard, l'activité luciférase est évaluée sur les extraits cellulaires en mettant en oeuvre un kit commercialisé par Proméga ("Luciferase Assay System") et, pour la mesure des échantillons, un compteur à scintillation (LS 50000 TD, Beckman). Le dosage s'avère positif pour 10 des clones évalués, ce qui indique qu'ils produisent un produit tTA fonctionnel capable de reconnaître les séquences tet O et d'activer l'expression des gènes placés sous leur contrôle.

### 2. Constitution d'un adénovirus dont l'expression des gènes E4 est inductible par le tTA.

Comme cela a été mentionné précédemment, l'expression des gènes codant pour les ORFs 6 et 7 de la région E4 est suffisante pour assurer la replication virale sans nécessité de complémentation. Cet exemple décrit la construction d'un vecteur adénoviral délété des régions E1 et E3 et d'une partie de E4, à l'exception des séquences codant pour les ORFs 6 et 7 dont l'expression est placée sous le contrôle de tet O et, de ce fait, stimulée en présence du tTA.

Le vecteur pUHD10-3 provient du clonage dans le plasmide pBR322 d'un fragment *Xho*I*-Eco*RI portant 7 copies tet O et le promoteur minimal CMV (position -53 par rapport au site d'initiation de la transcription ; voir Gossen et Bujard, 1992, *supra*). Après digestion par *Bam*HI (site situé en aval de la région promotrice), on insère le fragment *Bgl*II*-Bam*HI obtenu de pTG1653 et porteur des séquences codant pour les ORFs 6 et 7 pour donner pTG4658.

Le vecteur pTG4664 résulte du clonage du fragment adénoviral *Kpn*I*-Hpa*I (nucléotides 25 838 à 32 004) entre les sites *Kpn*I*-Sma*I d'un p poly II (Lathe et al., 1987, *supra*) dans lequel on avait au préalable supprimé le site *Bgl*II. Puis on effectue la délétion d'une grande partie de E3 (nucléotides 27 871 à 30 748) par digestion enzymatique (*Hpa*I-*Hind*III).

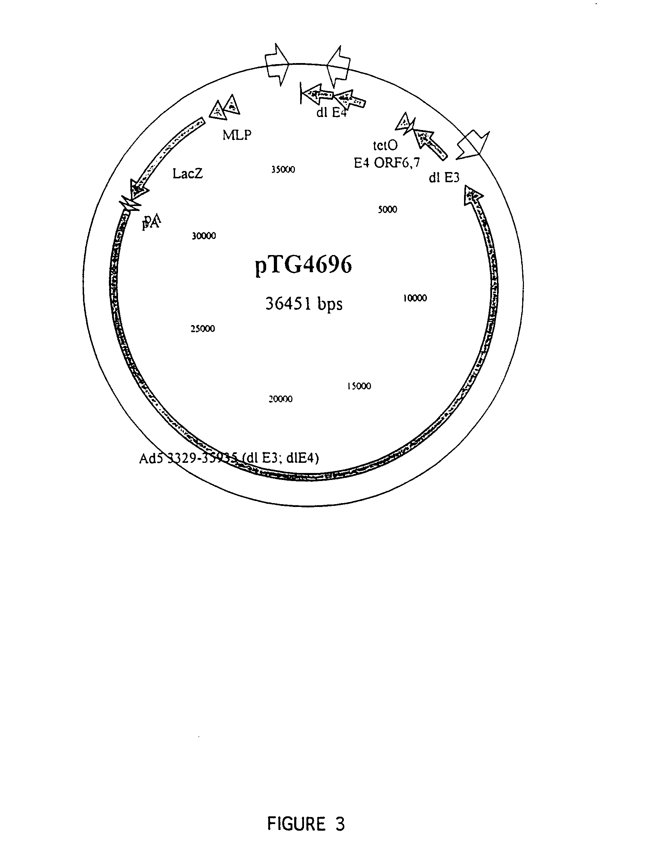

Le fragment *Xho*I*-Hpa*I porteur de la cassette d'expression régulée des ORFs 6 et 7 est isolé de pTG4658 et inséré après action de la Klenow dans le vecteur pTG4664 digéré par *Bgl*II et traité par la Klenow pour donner pTG4668 et pTG4669 selon l'orientation de la cassette. Celle-ci est introduite dans un vecteur adénoviral par recombinaison homologue. On utilise à cet effet le vecteur recombinant pTG4663 qui dérive d'un vecteur de seconde génération (délété des régions E1 et E4 essentielles à la replication et de la région E3 non-essentielle (délétion des nucléotides 27 871 à 30 748) ; décrit à l'exemple 4 de la demande internationale WO94/28152) dans lequel on a cloné la cassette d'expression "promoteur MLP-gène LacZ pA SV40" à la place de la région E1. Les cellules *E. coli* sont co-transformées par pTG4668 ou pTG4669 clivé par *Hind*III et le vecteur viral pTG4663 linéarisé par *Spe*I pour générer pTG4696 (Figure 3) et pTG4697.

D'autres vecteurs adénoviraux de type similaire peuvent être générés. On peut, par exemple, envisager de placer les gènes de la région E2 sous le contrôle des séquences tet O. L'homme du métier connait les techniques de biologie moléculaire qui permettent de remplacer le promoteur E2 par un promoteur régulable tel que celui isolé du vecteur pUHD10-3 ou d'éliminer les séquences en amont de la TATA box du promoteur E2 et d'introduire à leur place une ou plusieurs copies (de préférence 7) de séquences tet O. Il est également possible de construire des vecteurs régulés à plusieurs niveaux, par exemple par insertion de tet O en amont de la TATA box des promoteurs dirigeant l'expression des gènes viraux de E2, E4 et/ou MLP.

### 3. Génération de particules virales.

Le vecteur viral pTG4696 ou pTG4697 est transfecté dans l'un des 10 clones producteurs de tTA de l'exemple 1.1. Les cellules sont capables de complémenter la fonction E1 et de produire du tTA activant l'expression des ORFs 6 et 7, ce qui permet la formation de particules virales. On constitue un stock qui permet ensuite d'infecter les cellules cibles à une m.o.i. (multiplicité d'infection) déterminée susceptible de varier de 0,1 à 100.

### EXEMPLE 2 : Constitution de la lignée de complémentation 4677 pour les fonctions E1 et E4, l'expression de E4 étant inductible par le tTA.

Cet exemple a trait à une lignée de complémentation générée par transfection des cellules 293 par un vecteur d'expression des ORFs 6 et 7 de E4 placés sous le contrôle d'un promoteur minimal précédé de 7 copies de tet O. Les cellules transfectées sont capables de complémenter les fonctions E1 de manière constitutive et E4 de manière inductible par le tTA. Celui-ci peut être porté par un vecteur adénoviral défectif ou un vecteur d'expression classique.

### 1. Constitution de la lignée de complémentation.

Le fragment *Xho*I*-Bam*HI de pTG4658 (portant la cassette "tet O-promoteur minimal CMV-ORFs 6 et 7") est inséré entre les sites *Sal*I et *Bgl*II du vecteur de sélection pTG6529, pour donner pTG4677. 60 clones résistants ont été générés après transfection dans la lignée 293 et sélection par la puromycine. On peut déterminer les clones présentant une capacité optimale de complémentation de différentes façons.

Une première méthode consiste à transfecter de manière transitoire le plasmide pUHD15-1 exprimant de manière constitutive le tTA. Une analyse par Western blot des extraits cellulaires à l'aide d'anticorps appropriés permettra d'évaluer la quantité de polypeptides ORFs 6 et/ou 7 produits dans ces clones et donc leur capacité à trans-complémenter la fonction adénovirale E4. Selon une autre méthode, on peut employer un adénovirus défectif exprimant le tTA. Cette méthode est détaillée ci-après.

### 2. Constitution d'un adénovirus défectif exprimant le tTA d'une manière constitutive.

Deux séries de constructions ont été réalisées .pTG4682 correspondant au génome adénoviral délété de l'essentiel des régions E1 et E3 et comportant à la place de la région E1, une cassette d'expression constitutive du tTA (promoteur CMV). pTG4683 est en outre délété de la région E4.

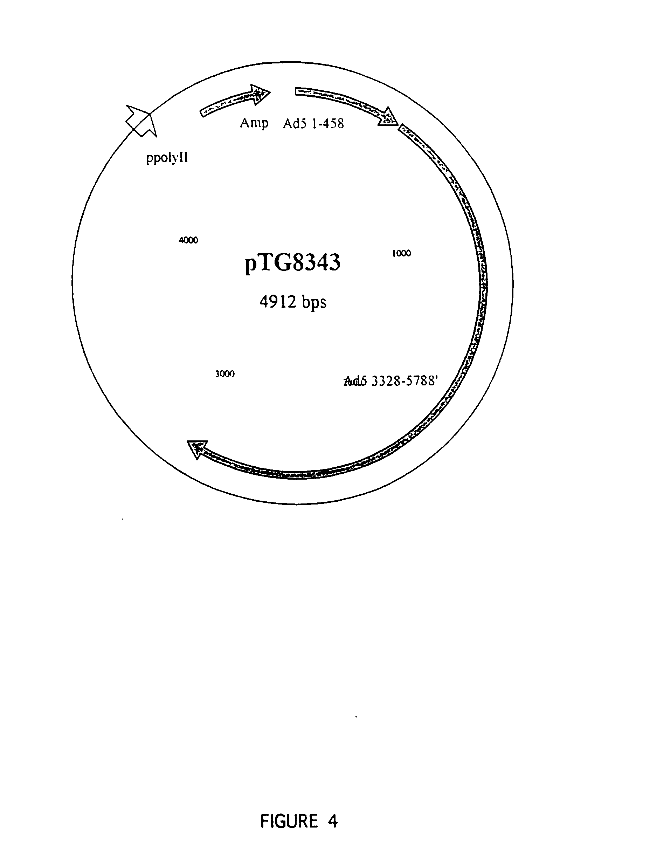

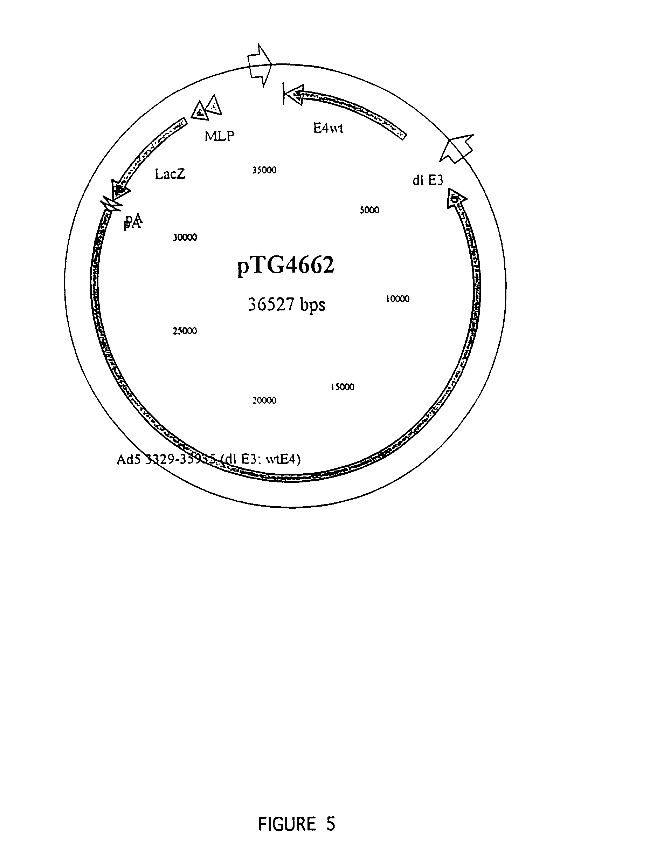

On part du vecteur pTG8343, lequel provient de l'insertion dans p poly II (Lathe et al., 1987, *supra*) d'une partie de l'extrémité 5' du génome adénoviral, à savoir les séquences s'étendant des nucléotides 1 à 458 et 3328 à 5788 (Figure 4). Après digestion de pTG8343 par *Bgl*II et traitement par la Klenow, celui-ci est mis en ligation avec le fragment *Ssp*I*-Hpa*I de pUHD15-1 portant le promoteur CMV suivi des séquences tTA. On obtient pTG4674 destiné à permettre l'insertion de la cassette du tTA par recombinaison homologue avec un vecteur adénoviral comprenant des séquences homologues. A cet effet, on choisit le vecteur pTG4662 (Figure 5) qui comporte l'ITR 5' et les séquences d'encapsidation de l'Ad5 (nt 1 à 458), une cassette d'expression du gène LacZ à la place de la région E1 et les séquences adénovirales restantes délétées de la région E3 (nucléotides 3329 à 27870 et 30749 à 35935).

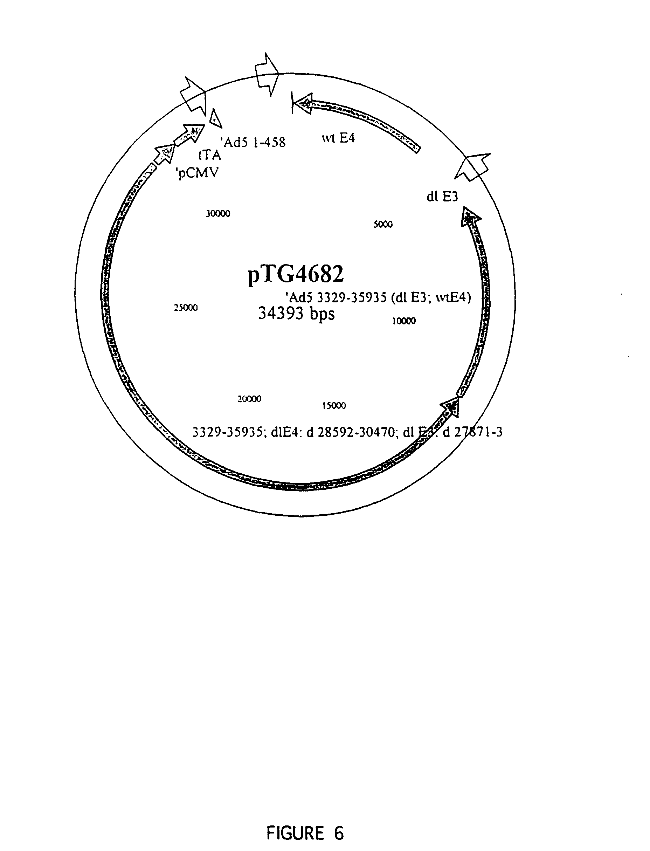

La souche *E. coli* BJ est co-transformée avec les vecteurs pTG4662 linéarisé par *Cla*I et pTG4674 clivé par *Sgr*AI et *Bst*EII, pour obtenir pTG4682 (Figure 6). Cet evènement de recombinaison homologue permet un échange de la cassette LacZ de pTG4662 contre celle du tTA portée par le fragment issu de pTG4674.

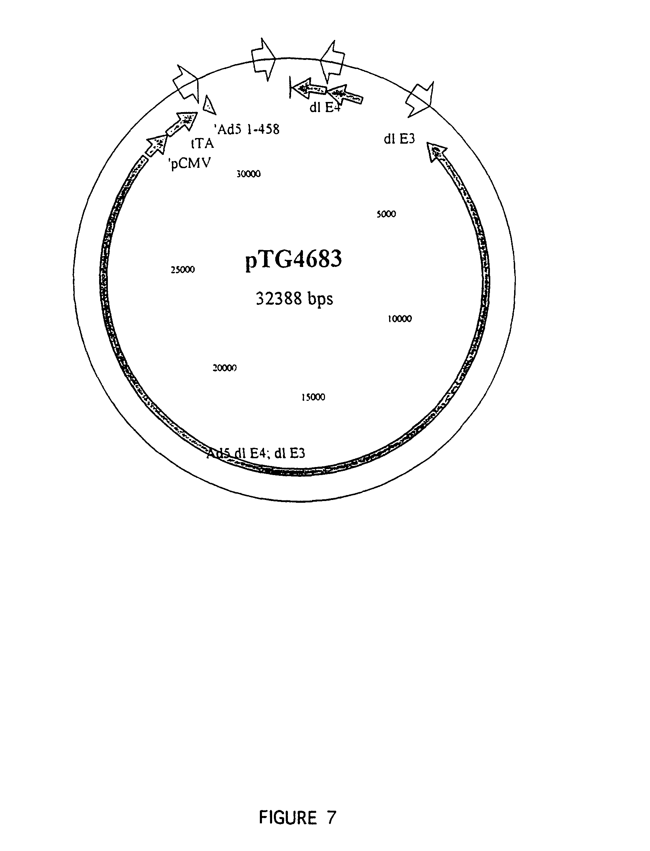

Le vecteur pTG4683 (Figure 7) est obtenu selon la même technologie par recombinaison homologue *in vitro* entre pTG4663 clivé par *Cla*I et pTG4674 digéré par *Sgr*AI et *Bst*EII. Le vecteur pTG4663 est similaire à pTG4662 à la différence qu'il est délété de l'essentiel de la région E4 (nucléotides 32994 à 34998).

### 3. Constitution d'un adénovirus défectif exprimant le tTA d'une manière inductible.

On a recours pour contrôler l'expression des séquences tTA, au promoteur minimal CMV comportant en 5' 7 copies de séquences tet O, ce qui rend le système autoinductible. En effet, la production de quelques molécules de tTA à partir du promoteur non induit va permettre d'activer le système. Comme avant, on génère deux vecteurs adénoviraux pTG4684 et pTG4685 délétés ou non de la région E4.

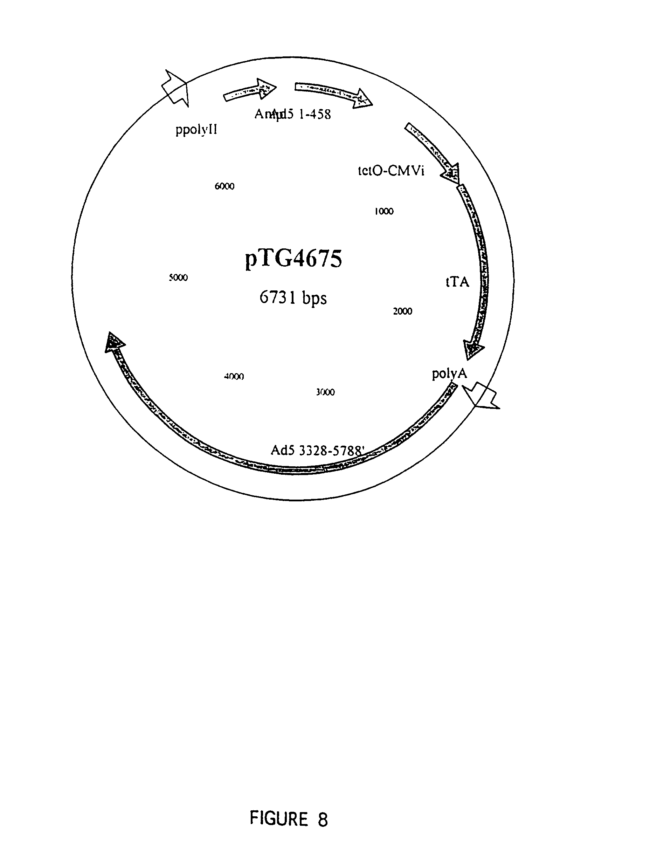

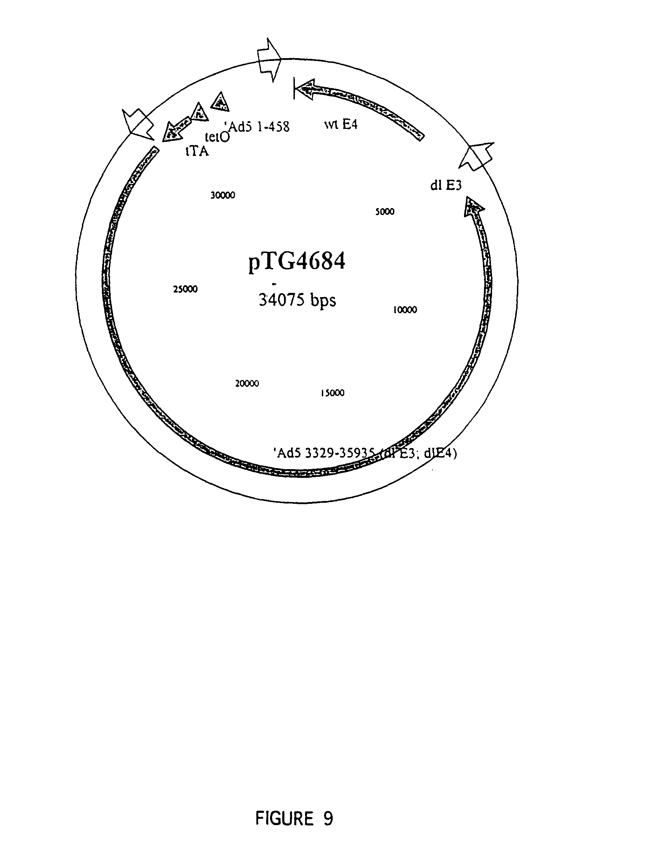

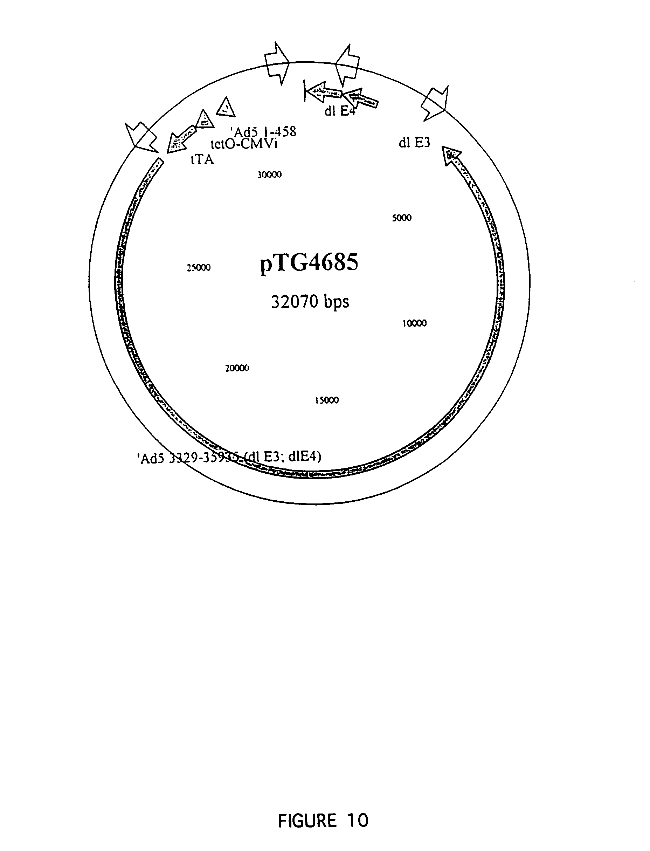

Le promoteur CMV de pUHD15-1 est échangé contre son homologue régulable isolé de pUHD10-3 sous forme d'un fragment *Xho*I*-Eco*RI. On obtient pTG4659. La cassette est isolée de ce dernier par digestion par *Ssp*I et *Hpa*I et clonée dans le vecteur pTG8343 linéarisé par *Bgl*II et traité par la Klenow, pour donner pTG4675 (Figure 8). La souche *E. coli* BJ est ensuite co-transformée par le vecteur précédent traité par *Sgr*AI et *Bst*EII et pTG4662 ou pTG4663 linéarisé par l'enzyme *Cla*I, afin de générer par recombinaison homologue les vecteurs viraux pTG4684 (Figure 9) et pTG4685 (Figure 10).

Les particules virales peuvent être obtenues par simple transfection d'une lignée cellulaire appropriée comme celle de l'exemple 2.1 en absence de tétracycline (l'addition de l'antibiotique dans le milieu de culture ayant un effet inhibiteur sur la transcription du tTA et des ORFs 6 et 7 régulée par les séquences tet O).

### 4. Tests fonctionnels de microinfection et de microtitration.

Les expériences sont réalisées en parallèle sur les cellules 293 complémentant la fonction E1 et les cellules 1653 complémentant les fonctions E1 et E4. Les cellules sont réparties dans une plaque de culture à raison d'environ 5 x 10⁴ cellules par puit. Elles sont ensuite transfectées par les vecteurs à tester, pTG4682 à 4685. On évalue leur capacité à former des plages dans les deux types cellulaires (suivant leur vitesse d'apparition) ainsi que leur taille. A titre de témoins, on utilise les vecteurs recombinants pTG4662 et pTG4663 qui correspondent respectivement à des vecteurs adénoviraux de première génération (délétion de E1 et E3) et de seconde génération (délétion supplémentaire de E4) et qui n'expriment pas le tTA.

La génération de particules virales à partir de la construction pTG4662 est facilement détectable et ceci dans les deux lignées cellulaires (formation de grandes plages apparaissant rapidement). Quant à pTG4663, il ne peut être propagé que dans les cellules 1653 complémentant E1 et E4 et donne de petites plages qui apparaissent tardivement.

En ce qui concerne les vecteurs pTG4682 et pTG4684, leur transfection dans les cellules 293 et 1653 est rapidement suivie de la formation de grandes plages facilement repérables (comportement similaire au témoin pTG4662). Les vecteurs pTG4683 et pTG4685 délétés de la région E4 se comportent d'une manière comparable à pTG4663 : dans la lignée 1653 les particules virales apparaissent lentement en formant des plages de taille réduite alors que dans la lignée 293 on ne détecte pas de plages. Ces données semblent indiquer que l'expression du tTA n'est pas préjudiciable à la propagation virale ni toxique pour la croissance cellulaire.

Comme indiqué précédemment, on peut appliquer cette technologie à la lignée de l'exemple 1 (293/pTG4673) et des vecteurs pTG4696 et pTG4697.

### EXEMPLE 3 : Constitution de la lignée de complémentation 5606 pour les fonctions E1 et E4 dans laquelle l'expression de E4 est inductible par le tTA produit par la lignée.

Cet exemple décrit une lignée cellulaire de complémentation permettant la propagation d'adénovirus déficient E1⁻ et E4⁻ obtenue par transfection des cellules 293 par le vecteur pTG5606 portant outre le gène de sélection *pac*, les séquences codant pour les ORFs 6/7 et pour le tTA placées sous le contrôle du promoteur désigné ci-après pCMV* correspondant au promoteur minimal CMV (position -53 par rapport au site d'initiation de la transcription) muni en 5' de 7 séquences consécutives tetO. Ce système autoinductible permet tout d'abord la production de quelques molécules de tTA à partir du promoteur CMV minimal qui pourront agir positivement sur les séquences tet O et amplifier leur propre synthèse et celle des produits ORF6/7 en quantité suffisante pour une complémentation efficace des vecteurs défectifs.

### 1. Construction du plasmide pTG5606

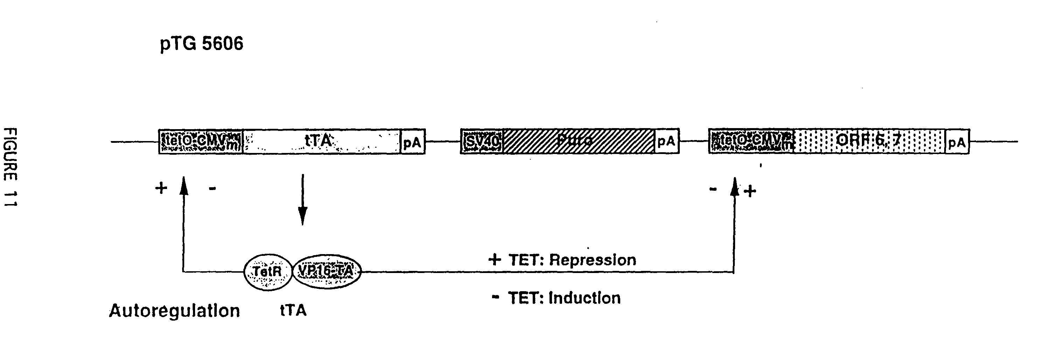

On commence par construire le vecteur pTG4688 qui résulte du clonage de la cassette (promoteur/enhancer CMV- tTA) purifiée de pTG4673 (exemple 1.1) clivé par *Hpa*I et *Pvu*I dans le vecteur pTG4677 (exemple 2.1) digéré par ces mêmes enzymes. Le vecteur pTG5606 est obtenu du vecteur précédent par remplacement du promoteur/enhancer CMV par le promoteur régulable pCMV*. Pour ce faire, pTG4688 est linéarisé par les enzymes *Sca*I*-Xba*I avant d'insèrer le fragment *Sca*I*-Xba*I portant pCMV* isolé de pTG4659. Le plasmide pTG5606 ainsi généré (Figure 11) comprenant les trois cassettes d'expression suivantes :
une première cassette composée du promoteur pCMV*, des séquences codant pour le trans-activateur tTA et des séquences polyA du virus SV40,
une deuxième cassette composée du gène de résistance à la puromycine (gène *pac*) sous le contrôle du promoteur précoce et des séquences pA du virus SV40, et
une troisième cassette composée du promoteur pCMV* suivi des séquences codant pour les ORFs 6/7 de l'adénovirus 5 munies de leur propre signal polyA.

### 2. Génération de la lignée de complémentation 5606.

4x10⁶ cellules 293 (ATCC CRL1573) sont mises en culture dans du milieu DMEM (Dubelco's modified Eagle's medium) en présence de FCS 10%. Aux environs de 70 à 80% de confluence, elles sont réparties en plusieurs boîtes puis sont transfectées par 10 µg ou 20 µg de plasmide pTG5606 (kit de transfection Gibco-BRL ; ref 530-8120SA) . Après 48 heures, les cellules sont cultivées en milieu sélectif (Puromycine 0,7 µg/ml pendant la première semaine et ensuite 1 µg/ml). Les clones résistants sont amplifiés dans le milieu sélectif précédent eventuellement en présence de tétracycline. Ce dernier exerce un effet répresseur sur les séquences tet O et son ajout a pour but de réduire la synthèse des produits d'expression des ORFs 6/7 qui pourrait s'avérer cytotoxique et entraîner la mort cellulaire. Ainsi, trois lots ont été constitués selon la concentration de tétracycline contenue dans le milieu, respectivement 0µg/ml , 1µg/ml et 5µg/ml. De nombreux clones apparaissent quelles que soient les quantités d'ADN transfecté et les conditions de culture.

Les clones co-exprimant les gènes E1 et E4 ont été criblés par une technique de microinfection et microtitration. Une centaine de clones 5606 est infectée à une faible moi (multiplicité d'infection) par un vecteur adénoviral défectif E1- E4- (voir par exemple la demande internationale WO 94/28152). On observe une cytopathie 48 à 72 h post-infection pour 10 à 16% d'entre eux selon le lot dont ils proviennent. La cytopathie témoigne de la multiplication des virus doublement défectifs et reflète la capacité de trans-complémentation du clone. Le titre en particules virales infectieuses est estimé par une technique de titrage en micropuits sur cellule permissive. Pour ce faire, les particules virales sont récupérées des cultures infectées par trois cycles de congélation-décongélation et les surnageants viraux à titrer sont dilués en cascade à raison de 100 µl par puit et mis en présence de 4x10⁴ cellules 1653. Le titre viral est évalué grossièrement par observation de la cytopathie aux différentes dilutions. Les clones 5606 les plus producteurs (cytopathie à des dilutions élevées) sont retenus pour une étude plus précise du rendement en pfu/ml et en ifu/ml.

Les clones 5606 sélectionnés sont mis en culture (5x10⁵ cellules) et infectés à nouveau par un vecteur adénoviral E1- E4- . On peut utiliser par exemple les vecteurs AdTG8595 et AdTG4651 délétés des régions E1, E3 (nt 28592 à 30470) et E4 (nt 32994 à 34998) et comprenant à la place de E1 la cassette d'expression du gène d'intérêt respectivement promoteur MPL-LacZ et promoteur MLP-gène CFTR. Comme précédemment, on récupère les surnageants viraux et le titre en pfu/ml est déterminé par infection d'une lignée permissive (lignée 1653 ou le clone producteur 5606) par des dilutions appropriées de surnageant viral et comptage des plages de lyse en agar. Deux clones désignés #5-19/1 et #5-38 donnent des titres variant de 1 à 5x10⁹ pfu/ml (titration effectuée sur eux-mêmes). Le rendement en particules infectieuses (ifu/ml) est évalué en dénombrant les cellules infectées exprimant le gène d'intérêt. Ainsi, les surnageants viraux obtenus des clones 5606 infectés par AdTG8595 sont titrés sur eux-mêmes et 48 heures post-infection, les cellules adhérentes sont fixées (tampon PBS contenant 2% de formaldéhyde et 0,2% de glutaraldéhyde) et la production de la β-galactosidase révélée par le colorant X-Gal (1 mg/ml dans du tampon PBS additionné de K-ferricyanide 5 mM, K-ferrocyanide 5 mM et MgCl₂ 2mM). Les titres obtenus sont compris entre 10¹⁰ et 10¹¹ ifu/ml.

Enfin, on vérifie que l'expression des gènes viraux tardifs n'est pas altérée, ce qui pourrait conduire à un défaut d'assemblage des particules virales. La production de fibre et de penton est déterminée par Western blot sur les culots de cellules infectées récupérés après les cycles de congélation-décongélation. L'analyse est effectuée selon les techniques standards sur un aliquot d'extrait cellulaire correspondant à 3 µg en protéines à l'aide d'anticorps dirigés contre la fibre (Henry et al., 1994, J. Virol. *68*, 5239-5246) ou le penton-base (Boulanger et al., 1973, Eur. J. Biochem. 39, 37-42) puis d'anticorps anti lapin marqués à la péroxidase (Amersham, NA 934). La révélation a lieu en utilisant le kit de détection ECL (Amersham, RPN 2106). Les données indiquent que les adénovirus E1- E4-générés à partir des deux clones 5606 sont capables de produire les protéines tardives à un niveau approchant de celui obtenu dans les cellules 293 infectées par un virus E1⁻.

En conclusion, les clones 5606 #5-19/1 et #5-38 constituent des lignées de complémentation E1 et E4 capables d'amplifier les vecteurs doublement défectifs à un titre dépassant 1x10⁹ pfu/ml, titre compatible avec une production à grande échelle.

### EXEMPLE 4 : Constitution de la lignée de complémentation 9579 pour les fonctions E1 et E2A dans laquelle l'expression de E2A est inductible par le tTA celui-ci étant produit par la lignée.

Cet exemple décrit une lignée cellulaire de complémentation permettant la propagation d'adénovirus déficients E1⁻ et E2A⁻ obtenue par transfection des cellules 293 par le vecteur pTG9579 permettant une expression autoinductible de la protéine DBP selon le même principe qu'à l'exemple précédent.

### 1. Construction du vecteur pTG9579

Le vecteur pTG9579 est construit de la façon suivante :

On isole l'extrémité 3' du génome de l'adénovirus 5 à partir d'une préparation d'ADN génomique digéré par *Dra*I*-Bsm*BI. Le fragment correspondant est sous-cloné dans le site *Sma*I du vecteur pUC19 (Gibco BRL), pour donner le vecteur intermédiaire pTG9568. On indique que le codon STOP de la séquence DBP est détruit par le clivage par *Dra*I. Celui-ci est restauré par introduction entre les sites *Eco*RV et *Bam*HI de pTG9568 d'un fragment comportant la séquence correcte obtenue par amplification PCR à partir de la matrice génomique et des amorces adéquates. On obtient pTG9571.

Par ailleurs, le vecteur pUHD-10-3 est linéarisé par *Hind*III, traité à la Klenow et on insère la cassette d'expression du gène neo (Colbère -Garapin et al., 1981, J. Mol. Biol. *150*, 1-14) dirigée par le promoteur précoce et le signal pA de SV40. Le vecteur ainsi obtenu pTG9555 est linéarisé par *Xba*I puis soumis à l'action de la Klenow avant de cloner le fragment portant la séquence DBP isolée de pTG9571 après clivage par *Xho*I et *Eco*RI et traitement à la Klenow. Enfin, on introduit dans le site *Xho*I traité à la Klenow du vecteur pTG9577 généré à l'étape précédente, le fragment *Hpa*I*-Xho*I (Klenow) isolé de pTG4659 porteur de la cassette d'expression du gène tTA, pour donner pTG9579. Pour résumer, celui-ci comprend :
une première cassette composée du promoteur pCMV* suivi des séquences codant pour le trans-activateur tTA et des séquences polyA du virus SV40,
une deuxième cassette composée du gène neo de résistance à l'antibiotique G418 sous le contrôle du promoteur précoce et des séquences pA du virus SV40, et
une troisième cassette composée des séquences codant pour la protéine DBP de l'adénovirus 5 sous le contrôle du promoteur pCMV* et des séquences pA du virus SV40.

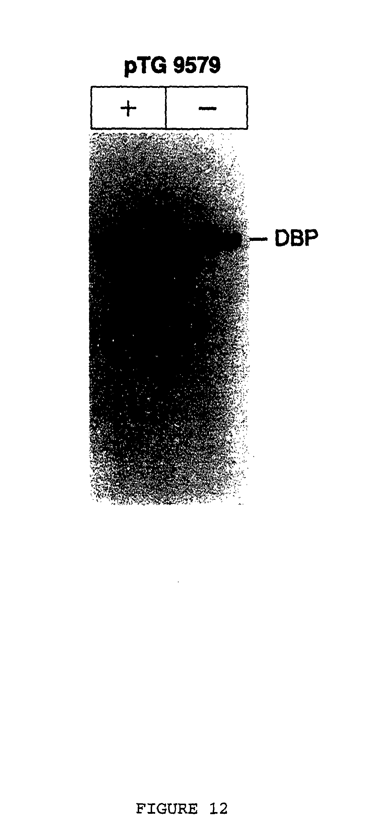

La production d'une protéine DBP fonctionnelle par pTG9579 peut être vérifiée par transfection transitoire dans les cellules 293. Brièvement, 1x10⁶ cellules sont transfectées par 5 µg de vecteur par la méthode au phosphate de calcium puis cultivées en présence (1 µg/ml) ou en absence de tétracycline. 4 jours après la transfection, les cellules sont récupérées et le culot cellulaire traité par 100 µl de tampon de lyse (50 mM Tris-HCL, 1 mM MgCl₂, 1 mM EDTA, 5 mM KCl, 150 mM NaCl et 1 % Triton X-100) afin de solubiliser les protéines. 7 µl d'extrait protéique sont repris dans un tampon de charge Tris Glycine contenant 5 % de p-mercaptoéthanol et soumis à une electophorèse PAGE 8-16 % en condition dénaturante. Après transfert sur membrane de nitrocellulose, la protéine DBP est révélée par Western blot à l'aide d'un anticorps spécifique de la protéine DBP adénovirale (Reich et al., 1983, Virology *128*, 480-484 ; par exemple l'anticorps monoclonal de souris α72KB 6-8) et un anticorps de mouton anti immunoglobuline de souris marqué à la péroxydase (Amersham ; NA 931). La révélation est effectuée par le kit ECL (Amersham, RPN 2106). Les résultats présentés à la Figure 12 montrent que la protéine DBP est produite dans les cellules 293 transfectées de manière transitoire par le vecteur pTG9579 et d'une manière régulée puisque l'expression est réduite en présence de tétracycline.

### 2. Genération de la lignée de cellulaire de complémentation 9579

On utilise 20 µg de plasmide pTG9579 pour transfecter 3x10⁶ cellules 293 (kit de transfection Gibco BRL ; ref 530-8120SA). Les clones sont sélectionnés en présence de 600 µg/ml de G418 et 1 µg/ml de tétracycline et testés pour leur capacité à produire la protéine DBP par la technique de Western blot décrite ci-avant. 20 % d'entre eux expriment des quantités détectables de DBP dont deux désignés #7-44 et #7-32 à un niveau proche de celui obtenu dans la lignée gmDBP6 en présence de dexaméthasone. Cette dernière constitue la lignée de référence pour la complémentation de le fonction E2A (Brough et al., 1992, Virol. *190*, 624-634). Elle dérive de cellules HeLa transfectées par une cassette d'expression comprenant les séquences codant pour la DBP dirigées par le promoteur MMTV inductible par le dexaméthasone.

Les clones DBP positifs sont mis en culture et infectés à une moi ≥ 1 par l'adénovirus H5dl802 défectif pour la fonction E2A (Rice and Klessig, 1985, J. Virol. *56*, 767-778). Deux jours après l'infection, les surnageants viraux sont recueillis après lyse des cellules par des cycles consécutifs de congélation-décongélation, dilués en cascade et titrés sur la lignée permissive gmDBP6. La présence de particules virales est observée dans les surnageants issus des clones #7-44 et #7-32 montrant leur capacité à complémenter la fonction E2A. Le même type d'expérience est réalisée avec le vecteur pTG9542 (E1⁻ (LacZ) E2A⁻ et E3⁻). Des particules infectieuses sont produites montrant que ces deux clones sont aptes à propager et amplifier des vecteurs doublement défectifs. De plus, celles-ci ne peuvent être propagées sur la lignée 293 confirmant le phénotype E2A⁻ et l'absence de révertants.

Des expériences d'amplification menées avec le clone #7-44 infecté par le virus AdTG9542 ont montré un facteur d'amplification d'environ 125 lorsque le titrage est effectué 4 jours post-infection.

## Revendications

1. Vecteur viral comprenant une unité d'expression fonctionnelle dans des cellules de complémentation et non fonctionnelle dans une cellule hôte contrôlant l'expression d'un ou plusieurs gènes viraux, **caractérisé en ce que** ladite unité d'expression comprend une ou plusieurs séquences de régulation permettant d'activer l'expression desdits gènes viraux en présence d'un inducteur et d'inhiber ladite expression en présence d'un répresseur.

2. Vecteur viral selon la revendication 1, **caractérisé en ce que** ladite unité d'expression comprend une ou plusieurs séquences de régulation dérivant d'un opéron bactérien.

3. Vecteur viral selon la revendication 2, **caractérisé en ce que** ladite unité d'expression comprend une ou plusieurs séquences de régulation dérivant de l'opéron tétracycline.

4. Vecteur viral selon la revendication 3, **caractérisé en ce que** ledit opéron tétracycline est codé par le transposon Tn10.

5. Vecteur viral selon la revendication 3 ou 4, **caractérisé en ce que** ladite séquence de régulation est la séquence tet 0.

6. Vecteur viral selon l'une des revendications 1 à 5, **caractérisé en ce que** ladite séquence de régulation est placée en amont de la TATA box du promoteur de ladite unité d'expression.

7. Vecteur viral selon l'une des revendications 1 à 5, **caractérisé en ce que** ladite séquence de régulation est placée en aval de la TATA box du promoteur de ladite unité d'expression.

8. Vecteur viral selon l'une des revendications 1 à 7, **caractérisé en ce que** ladite unité d'expression comprend de 1 à 20, de préférence de 1 à 10, et de manière tout à fait préférée de 1 à 7 séquences de régulation permettant d'activer l'expression desdits gènes viraux en présence d'un inducteur et d'inhiber ladite expression en présence d'un répresseur.

9. Vecteur viral selon l'une des revendications 6 à 8, **caractérisé en ce que** ledit promoteur est un promoteur minimal du virus CMV.

10. Vecteur viral selon l'une des revendications 1 à 9, **caractérisé en ce que** :
(i) ledit répresseur permettant d'inhiber ladite expression est sélectionné parmi le groupe consistant en l'antibiotique tétracycline, l'anhydro tétracycline et le répresseur tétracycline tet R, et
(ii) ledit inducteur permettant d'activer ladite expression consistant en une protéine résultant de la fusion entre le tet R et les 130 acides aminés C-terminaux du domaine d'activation de la protéine VP16 du virus simplex de l'herpès (tTA).

11. Vecteur viral selon l'une des revendications 1 à 10, dérivé d'un virus sélectionné parmi le virus de l'herpès, le cytomégalovirus, l'AAV (virus associé à l'adénovirus), la poxvirus et l'adénovirus.

12. Vecteur adénoviral selon la revendication 11, dérivé d'un adénovirus d'origine humaine, canine, aviaire, bovine, murine, ovine, porcine ou simienne ou encore d'un hybride comprenant des fragments de génome adénoviral de différentes origines.

13. Vecteur viral selon la revendication 11 ou 12, **caractérisé en ce qu'**il est défectif pour la replication.

14. Vecteur adénoviral selon la revendication 13, **caractérisé en ce qu'**il est au moins dépourvu de tout ou partie de la région E1 et, de façon optionnelle, de tout ou partie de la région E3.

15. Vecteur adénoviral selon l'une des revendications 12 à 14, **caractérisé en ce qu'**il comprend une ou plusieurs unité(s) d'expression comportant un ou plusieurs gène(s) adénoviraux des régions E2, E4 ou L1-L5.

16. Vecteur adénoviral selon la revendication 15, **caractérisé en ce qu'**il comprend une unité d'expression comportant une ou plusieurs séquence(s) de régulation dérivant de l'opéron tétracycline placée(s) en amont de la TATA box du promoteur et les cadres de lecture ouverts (ORFs) 6 et 7 de la région E4, de manière à ce que l'expression desdits cadres de lecture soit activable par un inducteur de type trans-activateur tétracycline (tTA) et répressible par la tétracycline.

17. Vecteur viral selon l'une des revendications 1 à 16, **caractérisé en ce qu'**il comprend une deuxième unité d'expression consistant en une séquence nucléotidique exogène placée sous le contrôle des éléments nécessaires à son expression dans la cellule hôte.

18. Vecteur viral selon la revendication 17, **caractérisé en ce que** la séquence nucléotidique exogène est sélectionnée parmi les gènes codant pour une cytokine, un récepteur cellulaire au nucléaire, un ligand, un facteur de coagulation, la protéine CFTR, l'insuline, la dystrophine, une hormone de croissance, une enzyme, un inhibiteur d'enzymes, un polypeptide à effet anti-tumoral, un polypeptide capable d'inhiber une infection bactérienne, parasitaire ou virale et, notamment le HIV, un anticorps, une toxine, une immunotoxine et un marqueur.

19. Vecteur viral selon l'une des revendications 17 et 18, **caractérisé en ce que** les éléments nécessaires à l'expression de ladite séquence nucléotidique exogène renferment un promoteur différent de celui inclus dans l'unité d'expression contrôlant l'expression du ou desdits gènes viraux.

20. Particule virale infectieuse comprenant un vecteur viral selon l'une des revendications 1 à 19.

21. Cellule hôte eucaryote comprenant un vecteur viral selon l'une des revendications 1 à 19 où une particule virale infectieuse selon la revendication 20.

22. Procédé de préparation d'une particule virale infectieuse selon la revendication 20, selon lequel :
(i) on introduit un vecteur viral selon l'une des revendications 1 à 19 dans une cellule de complémentation capable de complémenter *en trans* ledit vecteur viral pour obtenir une cellule de complémentation transfectée ;
(ii) on cultive ladite cellule de complémentation transfectée dans des conditions appropriées pour permettre l'expression des gènes viraux et la production de ladite particule virale infectieuse, et
(iii) on récupère ladite particule virale infectieuse dans la culture cellulaire.

23. Produit de combinaison comprenant :
(i) un vecteur viral selon l'une des revendications 1 à 19, une particule virale infectieuse selon la revendication 20 ou obtenue selon le procédé de la revendication 22, ou une cellule hôte eucaryote selon la revendication 21,
(ii)un répresseur capable d'inhiber l'expression desdits gènes viraux.

24. Produit de combinaison selon la revendication 23 **caractérisé en ce que** ledit répresseur est sélectionné parmi le groupe consistant en l'antibiotique tétracycline, l'anhydro tétracycline et le répresseur tétracycline tet R.

25. Composition pharmaceutique comprenant un vecteur viral selon l'une des revendications 1 à 19, une particule virale infectieuse selon la revendication 20 ou obtenue selon le procédé de la revendication 22, une cellule hôte eucaryote selon la revendication 21, ou un produit de combinaison selon la revendication 23 ou 24, en association avec un support acceptable d'un point de vue pharmaceutique.

26. Utilisation d'un vecteur viral selon l'une des revendications 1 à 19, d'une particule virale infectieuse selon la revendication 20 ou obtenue selon le procédé de la revendication 22, d'une cellule hôte eucaryote selon la revendication 21, d'un produit de combinaison selon la revendication 23 ou 24, ou d'une composition selon la revendication 25 pour la préparation d'un médicament destiné à la mise en oeuvre d'un traitement du corps humain ou animal par thérapie génique.

27. Utilisation selon la revendication 26 en association avec un répresseur.

28. Utilisation selon la revendication 27, **caractérisée en ce que** ledit répresseur est sélectionné parmi le groupe consistant en l'antibiotique tétracycline, l'anhydro tétracycline et le répresseur tétracycline tet R.

## Patentansprüche

1. Viraler Vektor, umfassend eine in Komplementationszellen funktionelle und in einer Wirtszelle, welche die Expression eines oder mehrerer viraler Gene kontrolliert, nicht funktionelle Expressionseinheit, **dadurch gekennzeichnet, daß** die Expressionseinheit eine oder mehrere Regulationssequenzen umfaßt, welche die Aktivierung der Expression der viralen Gene in Anwesenheit eines Induktors und die Inhibierung der Expression in Gegenwart eines Repressors gestatten.

2. Viraler Vektor nach Anspruch 1, **dadurch gekennzeichnet, daß** die Expressionseinheit eine oder mehrere Regulationssequenzen umfaßt, die von einem Bakterien-Operon abstammen.

3. Viraler Vektor nach Anspruch 2, **dadurch gekennzeichnet, daß** die Expressionseinheit eine oder mehrere Regulationssequenzen umfaßt, die vom Tetracyclin-Operon abstammen.

4. Viraler Vektor nach Anspruch 3, **dadurch gekennzeichnet, daß** das Tetracyclin-Operon durch das Transposon Tn 10 codiert ist.

5. Viraler Vektor nach Anspruch 3 oder 4, **dadurch gekennzeichnet, daß** die Regulationssequenz die Sequenz tet O ist.

6. Viraler Vektor nach einem der Ansprüche 1 bis 5, **dadurch gekennzeichnet, daß** die Regulationssequenz stromaufwärts bezüglich der TATA-Box des Promotors der Expressionseinheit angeordnet ist.

7. Viraler Vektor nach einem der Ansprüche 1 bis 5, **dadurch gekennzeichnet, daß** die Regulationssequenz stromabwärts bezüglich der TATA-Box des Promotors der Expressionseinheit angeordnet ist.

8. Viraler Vektor nach einem der Ansprüche 1 bis 7, **dadurch gekennzeichnet, daß** die Expressionseinheit 1 bis 20, vorzugsweise 1 bis 10 und ganz besonders bevorzugt 1 bis 7 Regulationssequenzen umfaßt, welche die Aktivierung der Expression der viralen Gene in Anwesenheit eines Induktors und die Inhibierung der Expression in Gegenwart eines Repressors gestatten.

9. Viraler Vektor nach einem der Ansprüche 6 bis 8, **dadurch gekennzeichnet, daß** der Promotor ein minimaler Promotor des Virus CMV ist.

10. Viraler Vektor nach einem der Ansprüche 1 bis 9, **dadurch gekennzeichnet, daß**:
i) der Repressor, der die Inhibierung der Expression gestattet, ausgewählt ist aus der Gruppe, die aus dem Antibiotikum Tetracyclin, Anhydrotetracyclin und dem Tetracyclin-Repressor tet R besteht, und
ii) der Induktor, welche die Aktivierung der Expression gestattet, aus einem Protein besteht, das aus der Fusion zwischen tet R und den 130 C-terminalen Aminosäuren der Aktivierungsdomäne des Proteins VP16 des Herpes simplex-Virus (tTA) resultiert.

11. Viraler Vektor nach einem der Ansprüche 1 bis 10, der von einem Virus abstammt, das aus dem Herpes-Virus, dem Cytomegalovirus, AAV (Adenovirus-assoziiertem Virus), dem Pockenvirus und dem Adenovirus ausgewählt ist.

12. Adenoviraler Vektor nach Anspruch 11, abstammend von einem Adenovirus menschlichen, Hunde-, Vogel-, Rinder-, Maus-, Schafs-, Schweine- oder Affen-Ursprungs oder auch von einem Hybrid, das Fragmente des adenoviralen Genoms aus unterschiedlichen Ursprüngen umfaßt.

13. Viraler Vektor nach Anspruch 11 oder 12, **dadurch gekennzeichnet, daß** replikationsdefizient ist.

14. Adenoviraler Vektor nach Anspruch 13, **dadurch gekennzeichnet, daß** ihm mindestens die ganze oder ein Teil der Region E1 und fakultativ die ganze oder ein Teil der Region E3 fehlt.

15. Adenoviraler Vektor nach einem der Ansprüche 12 bis 14, **dadurch gekennzeichnet, daß** er eine oder mehrere Expressionseinheit(en) umfaßt, welche ein oder mehrere adenovirale(s) Gen(e) der Regionen E2, E4 oder L1-L5 umfaßt.

16. Adenoviraler Vektor nach Anspruch 15, **dadurch gekennzeichnet, daß** er eine Expressionseinheit umfaßt, die eine oder mehrere Regulationssequenz(en), die vom Tetracyclin-Operon abstammen, stromaufwärts bezüglich der TATA-Box des Promotors und die offenen Leseraster (ORFs) 6 und 7 der Region E4 auf solche Weise umfaßt, **daß** die Expression der Leseraster durch einen Induktor vom Typ trans-Tetracyclin-Aktivator (tTA) aktivierbar ist und durch Tetracyclin reprimierbar ist.

17. Viraler Vektor nach einem der Ansprüche 1 bis 16, **dadurch gekennzeichnet, daß** er eine zweite Expressionseinheit umfaßt, welche aus einer exogenen Nukleotidsequenz besteht, die unter der Kontrolle der Elemente angeordnet ist, die für ihre Expression in der Wirtszelle notwendig sind.

18. Viraler Vektor nach Anspruch 17, **dadurch gekennzeichnet, daß** die exogene Nukleotidsequenz aus Genen ausgewählt ist, die für ein Cytokin, einen zellulären oder Kern-Rezeptor, einen Liganden, einen Koagulationsfaktor, das Protein CFTR, Insulin, Dystrophin, ein Wachstumshormon, ein Enzym, einen Inhibitor von Enzymen, ein Polypeptid mit Anti-Tumorwirkung, ein Polypeptid, das eine bakterielle, parasitäre oder virale und insbesondere die HIV-Infektion hemmen kann, einen Antikörper, ein Toxin, ein Immunotoxin und einen Marker codieren.

19. Viraler Vektor nach einem der Ansprüche 17 und 18, **dadurch gekennzeichnet, daß** die zur Expression der exogenen Nukleotidsequenz notwendigen Elemente einen Promotor einschließen, der von demjenigen verschieden ist, der in der Expressionseinheit eingeschlossen ist, welche die Expression des oder der viralen Gene kontrolliert.

20. Infektiöses virales Teilchen, das einen viralen Vektor gemäß einem der Ansprüche 1 bis 19 umfaßt.

21. Eukaryontische Wirtszelle, umfassend einen viralen Vektor nach einem der Ansprüche 1 bis 19 oder ein infektiöses virales Teilchen nach Anspruch 20.

22. Verfahren zur Herstellung eines infektiösen viralen Teilchens gemäß Anspruch 20, gemäß dem man:
i) einen viralen Vektor nach einem der Ansprüche 1 bis 19 in eine Komplementationszelle einführt, welche in der Lage ist, den viralen Vektor in trans zu komplementieren, um eine transfizierte Komplementationszelle zu erhalten;
ii) die transfizierte Komplementationszelle unter Bedingungen kultiviert, die geeignet sind, um die Expression der viralen Gene und die Produktion des infektiösen viralen Teilchens zu gestatten, und
iii) das infektiöse virale Teilchen in der Zellkultur gewinnt.

23. Kombinationsprodukt, umfassend:
i) einen viralen Vektor nach einem der Ansprüche 1 bis 19, ein infektiöses virales Teilchen nach Anspruch 20 oder erhalten gemäß dem Verfahren von Anspruch 22 oder eine eukaryontische Wirtszelle nach Anspruch 21,
ii) einen Repressor, der die Expression der viralen Gene inhibieren kann.

24. Kombinationsprodukt nach Anspruch 23, **dadurch gekennzeichnet, daß** der Repressor aus der Gruppe ausgewählt ist, die aus dem Antibiotikum Tetracyclin, Anhydrotetracyclin und dem Tetracyclin-Repressor tet R besteht.

25. Pharmazeutische Zusammensetzung, umfassend einen viralen Vektor nach einem der Ansprüche 1 bis 19, ein infektiöses virales Teilchen nach Anspruch 20 oder erhalten nach dem Verfahren von Anspruch 22, eine eukaryontische Wirtszelle nach Anspruch 21 oder ein Kombinationsprodukt nach Anspruch 23 oder 24 in Verbindung mit einem unter pharmazeutischem Gesichtspunkt annehmbaren Träger.

26. Verwendung eines viralen Vektors nach einem der Ansprüche 1 bis 19, eines infektiösen viralen Teilchens nach Anspruch 20 oder erhalten gemäß dem Verfahren von Anspruch 22, einer eukaryontischen Wirtszelle nach Anspruch 21, eines Kombinationsprodukts nach Anspruch 23 oder 24 oder einer Zusammensetzung nach Anspruch 25 für die Herstellung eines Medikaments, welches dazu bestimmt ist, eine Behandlung des menschlichen oder tierischen Körpers durch Gentherapie zu bewirken.

27. Verwendung nach Anspruch 26 in Verbindung mit einem Repressor.

28. Verwendung nach Anspruch 27, **dadurch gekennzeichnet, daß** der Repressor aus der Gruppe ausgewählt ist, die aus dem Antibiotikum Tetracyclin, Anhydrotetracyclin und dem Tetracyclin-Repressor tet R besteht.

## Claims

1. Viral vector comprising an expression unit which is functional in complementation cells and nonfunctional in a host cell controlling the expression of one or more viral genes, **characterized in that** the said expression unit comprises one or more regulatory sequences enabling the expression of the said viral genes to be activated in the presence of an inducer and the said expression to be inhibited in the presence of a repressor.

2. Viral vector according to Claim 1, **characterized in that** the said expression unit comprises one or more regulatory sequences derived from a bacterial operon.

3. Viral vector according to Claim 2, **characterized in that** the said expression unit comprises one or more regulatory sequences derived from the tetracycline operon.

4. Viral vector according to Claim 3, **characterized in that** the said tetracycline operon is coded for by transposon Tn10.

5. Viral vector according to Claim 3 or 4, **characterized in that** the said regulatory sequence is the tet 0 sequence.

6. Viral vector according to one of Claims 1 to 5, **characterized in that** the said regulatory sequence is placed upstream of the TATA box of the promoter of the said expression unit.

7. Viral vector according to one of Claims 1 to 5, **characterized in that** the said regulatory sequence is placed downstream of the TATA box of the promoter of the said expression unit.

8. Viral vector according to one of Claims 1 to 7, **characterized in that** the said the expression unit comprises from 1 to 20, preferably from 1 to 10, and most preferably from 1 to 7 regulatory sequences enabling the expression of the said viral genes to be activated in the presence of an inducer and the said expression to be inhibited in the presence of a repressor.

9. Viral vector according to one of Claims 6 to 8, **characterized in that** the said promoter is a minimal promoter of the CMV virus.

10. Viral vector according to one of Claims 1 to 9, **characterized in that**:
(i) the said repressor enabling the said expression to be inhibited is selected from the group consisting of the antibiotic tetracycline, anhydrotetracycline and the tetracycline repressor tet R, and
(ii) the said inducer enabling the said expression to be activated consisting of a protein resulting from the fusion between tet R and the 130 C-terminal amino acids of the activating domain of the VP16 protein of the herpes simplex virus (tTA).

11. Viral vector according to one of Claims 1 to 10, derived from a virus selected from the herpesvirus, cytomegalovirus, AAV (adeno-associated virus), poxvirus and adenovirus.

12. Adenoviral vector according to Claim 11, derived from an adenovirus of human, canine, avian, bovine, murine, ovine, porcine or simian origin, or alternatively from a hybrid comprising adenoviral genome fragments of different origins.

13. Viral vector according to Claim 11 or 12, **characterized in that** it is defective for replication.

14. Adenoviral vector according to Claim 13, **characterized in that** it lacks at least all or part of the E1 region and, optionally, all or part of the E3 region.

15. Adenoviral vector according to one of Claims 12 to 14, **characterized in that** it comprises one or more expression unit(s) containing one or more adenoviral gene(s) of the E2, E4 or L1-L5 regions.

16. Adenoviral vector according to Claim 15, **characterized in that** it comprises an expression unit containing one or more regulatory sequence(s) derived from the tetracycline operon, placed upstream of the TATA box of the promoter and open reading frames (ORFs) 6 and 7 of the E4 region, so that the expression of the said reading frames is activable by an inducer of the tetracycline transactivator (tTA) type and repressible by tetracycline.

17. Viral vector according to one of Claims 1 to 16, **characterized in that** it comprises a second expression unit consisting of an exogenous nucleotide sequence placed under the control of the elements necessary for its expression in the host cell.

18. Viral vector according to Claim 17, **characterized in that** the exogenous nucleotide sequence is selected from the genes coding for a cytokine, a cell or nuclear receptor, a ligand, a coagulation factor, the CFTR protein, insulin, dystrophin, a growth hormone, an enzyme, an enzyme inhibitor, a polypeptide having an antitumour effect, a polypeptide capable of inhibiting a bacterial, parasitic or viral infection, and in particular HIV, an antibody, a toxin, an immunotoxin and a marker.

19. Viral vector according to either of Claims 17 and 18, **characterized in that** the elements necessary for the expression of the said exogenous nucleotide sequence contain a promoter different from that included in the expression unit controlling the expression of the said viral gene(s).

20. Infectious viral particle comprising a viral vector according to one of Claims 1 to 19.

21. Eukaryotic host cell comprising a viral vector according to one of Claims 1 to 19 or an infectious viral particle according to Claim 20.

22. Method of preparation of an infectious viral particle according to Claim 20, according to which:
(i) a viral vector according to one of Claims 1 to 19 is introduced into a complementation cell capable of complementing *in trans* the said viral vector, to obtain a transfected complementation cell;
(ii) the said transfected complementation cell is cultured under suitable conditions to permit the expression of the viral genes and the production of the said infectious viral particle; and
(iii) the said infectious viral particle is recovered in the cell culture.

23. Combination product comprising:
(i) a viral vector according to one of Claims 1 to 19, an infectious viral particle according to Claim 20 or obtained according to the method according to Claim 22, or a eukaryotic host cell according to Claim 21.
(ii) a repressor capable of inhibiting the expression of the said viral genes.

24. Combination product according to Claim 23, **characterized in that** the said repressor is selected from the group consisting of the antibiotic tetracycline, anhydrotetracycline and the tetracycline repressor tet R.

25. Pharmaceutical composition comprising a viral vector according to one of Claims 1 to 19, an infectious viral particle according to Claim 20 or obtained according to the method of Claim 22, a eukaryotic host cell according to Claim 21, or a combination product according to Claim 23 or 24, in combination with a vehicle which is acceptable from a pharmaceutical standpoint.

26. Use of a viral vector according to one of Claims 1 to 19, of an infectious viral particle according to Claim 20 or obtained according to the method of Claim 22, of a eukaryotic host cell according to Claim 21, of a combination product according to Claim 23 or 24 or of a composition according to Claim 25, for the preparation of a medicinal product intended for treating of the human or animal body by gene therapy.

27. Use according to Claim 26, in combination with a repressor.

28. Use according to Claim 27, **characterized in that** the said repressor is selected from the group consisting of the antibiotic tetracycline, anhydrotetracycline and the tetracycline repressor tet R.
